# EUROPEAN PATENT SPECIFICATION

(11) **EP 3 342 443 B1**
(45) Date of publication and mention of the grant of the patent: **06.11.2019**
(21) Application number: 16207372.0
(22) Date of filing: 29.12.2016
(51) Int. Cl.: A61M 16/06, A61M 16/08, A61M 16/20, G01F 15/06, A61M 16/00

(54) **GAS FLOW INDICATOR DEVICE**
GASSTROMANZEIGEVORRICHTUNG
DISPOSITIF D'INDICATION DE DÉBIT DE GAZ

(43) Date of publication of application: 04.07.2018
(73) Proprietor: VPAS Group Pty Ltd., Victoria 3145 (AU)
(72) Inventor: Matusik, Matthew, Malvern East, Victoria 3145 (AU)
(74) Representative: Herzog IP Patentanwalts GmbH

(56) References cited:
- WO-A1-2014/026221
- DE-B- 1 176 885
- JP-A- 2016 017 756
- US-A1- 2006 266 133

## Description

### FIELD OF THE INVENTION

The present invention relates generally to gas monitoring device. More particularly, the present invention relates to gas flow indicator device for gas delivery devices or systems and gas supply conduits.

It will be convenient to hereinafter describe the invention in relation to gas flow indicator device for medical devices, systems or conduits that deliver breathing gas to an individual's airway. However, it is to be appreciated that the present invention is not limited solely to that use. For example, an alternative use for gas flow indicator device of the invention could include breathing gas delivery devices or systems and gas supply conduits for use in the aviation industry. Also, the gas flow indicator device of the invention could be used for other, non-breathing gas applications, and the invention should not be construed as limited to any one or more of the specific examples provided herein.

### BACKGROUND OF THE INVENTION

Any discussion of documents, devices, acts or knowledge in this specification is included to explain the context of the invention. It should not be taken as an admission that any of the material forms a part of the prior art base or the common general knowledge in the relevant art in Australia, Europe, New Zealand, the United States of America or elsewhere, on or before the priority date of the disclosure herein.

Unless stated otherwise, throughout the ensuing description, "gas" or "gases" refers to any suitable gas, mixture of gases, vapour or a gas/vapour mixture that, as such, can be monitored using the gas flow indicator device of the invention. A gas with which the flow indicator device can be used can entrain an aerosol. In the context of medical or other respiratory applications of the gas flow indicator device, "gas" refers to any suitable breathing gas, generally oxygen, a mixture of oxygen and inert gas and/or pharmacological agent, air and oxygen-enriched air. Similarly, the expression "gas delivery device", "gas delivery system" or "gas delivery conduit" refers to any suitable device, system and conduit for supplying gas to, or at, a desired location such as, in the context of a medical or other respiratory application, to or into an individual's airway. Examples of suitable device, devices, system or conduit include, but are not limited to face masks, mouth pieces, nasal cannulas and gas supply conduits. For non-spontaneous breathing applications suitable device, devices, systems and conduits may include manual resuscitator devices, such as, for example, bag valve masks and endotracheal tubes.

Supplemental gas is widely used in the medical field. For example, supplemental oxygen is used to assist or maintain safe blood oxygen levels for a patient. The duration of supply of supplemental oxygen varies, depending on the condition of the patient and/or the particular circumstance necessitating supplemental oxygen being administered. Common scenarios include patients having a cardiorespiratory disease or dysfunction and surgical/anaesthetic intervention that mandates supplementation of atmospheric air with higher concentrations of inspired oxygen in order to achieve normal oxygen tensions in the patient's blood. Failure to deliver supplemental oxygen can lead to risk of reduced arterial oxygen tension that, if not corrected, can contribute directly to increased morbidity and mortality.

Failure of supplemental gas delivery is an acknowledged and feared system risk in hospitals. To safeguard against this, more often than not there are multilevel complex alarms and flow sensors within the hospital's in-built gas piping circuitry, and/or at gas supply outlets provided throughout hospital facilities. In addition, anaesthetic machines, intensive care ventilators, or the likes, have mandatory flow sensors engineered into their design to detect and alert of gas supply failure.

The gas supply systems of most hospitals generally are monitored, from the source to the supply outlet. The same applies to complex anaesthetic machines and ventilators. However, the most commonly used, and often most simple, gas delivery devices, systems and conduits, are not provided with any effective gas flow indicator device. Hence, their use can lead to the failure of gas delivery going unnoticed. This risk is increased in situations involving gas supply from portable gas tanks or cylinders.

One of the most commonly used gas delivery devices for spontaneously breathing patients is the gas delivery mask, or oxygen mask. Sometimes called the "Hudson Mask", with reference to the early mask innovations of the Hudson Company, most such masks are made of a clear plastics material and include a body that is sized to seat over the nose and mouth of a patient. With conventional mask designs, gas is introduced through a gas inlet, and expiratory gases are vented from either around the side of the mask or through appropriately placed ventilation apertures. Gas is supplied to the gas inlet from a gas supply source, commonly by way of a length of clear plastic conduit. The gas supply source may be an in-built hospital supply source, or a gas tank or cylinder.

Oxygen masks usually are designed to increase a patient's inspired fraction of oxygen, such as from about 21% to about 40%. The oxygen flow rate required to achieve this is about 6 litres per minute ("6 L/min"). When oxygen flow into the mask fails the prescribed inspired oxygen concentration is not achieved. Of greater concern, the patient re-breathes their expired gases which can't be replenished satisfactorily by entrainment of air around the side of the mask, ultimately leading to the inspiration of a gas mixture with a low level of oxygen (less than 21% oxygen) and a risk of hypoxemia. Oxygen masks are not presently provided with any visual indicator confirming the presence of oxygen flow into the mask, or in the oxygen supply conduit proximate the mask. With the use of a mask, it is not inherently obvious to a medical practitioner, to a carer or even to the patient when there is an insufficient or complete lack of oxygen flow. A visual inspection of the oxygen delivery system (e.g. conduit and mask) will not generally indicate whether or not oxygen is or flowing. For this reason, medical practitioners often find it necessary to use audible cues as a means of identifying that there is a flow of oxygen. However, even putting ones ear or a stethoscope adjacent to a mask may sometimes still not make clear whether gas is flowing, and the medical practitioner still has no way of knowing whether oxygen is being supplied at the prescribed flow rate of oxygen, such as about 6 L/min.

For non-spontaneously breathing patients, one of the most commonly used manual resuscitator gas delivery devices is the bag valve mask or "BVM". Sometimes called the "AMBU" bag or mask, with reference to the proprietary name appointed by the inventors' of the original BVM, such devices consist of a flexible air chamber (the "bag") attached to a face mask or endotracheal tube via a shutter valve. When the mask is properly applied to a patient (or endotracheal tube is correctly inserted into the patient's trachea) and the "bag" is squeezed, the device forces air into the patient's lungs. When the bag is released, it self-inflates from its supply end, drawing in either ambient air or oxygen supplied by an oxygen supply source, while also allowing the patient's lungs to deflate to the ambient environment (but not to the "bag") by way of a one-way expired air valve. The BVM generally includes two inlet ports for drawing in ambient air or oxygen. When available, oxygen is supplied to one of the inlet ports from a gas supply source, commonly by way of a length of clear plastic conduit. The gas supply source may be an in-built hospital supply source, or a gas tank or cylinder. The other inlet port can then be used to draw in ambient air, or to connect a reservoir for catching unused oxygen between compressions of the "bag". In case oxygen flow is not sufficient to fill the "bag", the reservoir generally includes a one-way valve for drawing in ambient air to ensure that the BVM continues to supply at least ambient air to the patient.

BVMs are designed to deliver up to 100% inspired oxygen to a patient. With a loss of supplemental oxygen supply into the "bag", the BVM will continue to entrain ambient air (with an oxygen concentration of about 21%) with which to ventilate the patient. However, patients requiring the use of such manual resuscitator devices often have severely compromised respiratory function, which means that they require much higher inspired oxygen concentrations than that of ambient air. Therefore, any loss of supplemental oxygen supply can have catastrophic sequelae if undiagnosed. As with the case of the common oxygen mask described above, loss of oxygen supply to a BVM can be, and often is, missed as there is presently no visual flow indicator device provided at or proximate the BVM confirming supplemental oxygen inflow. Again, although the presence of a sound may indicate that gas is flowing, the medical practitioner still has no way of knowing whether the required flow rate of oxygen is present at the BVM.

Often gas tanks or cylinders are used to supply oxygen to masks or BVM's, most commonly in emergency, perioperative, critical care or transport scenarios. While some cylinders do have ball-type flow indicators at their supply outlets, the cylinders are often placed in positions in which they are visually obscured, such as under a patient's bed or transportation trolley, or placed sideways rendering the ball-type flow indicators inaccurate. Additionally, most cylinders do not have alarms in the event of cylinder oxygen supply running empty during use to indicate oxygen supply failure. Even more concerning is that newer designs of oxygen cylinders commonly no longer have ball-type or other flow indicator incorporated into their design. Hence, failure of supply of supplemental gas to gas delivery devices or systems is a real and likely problem.

A substantial advance in the art is provided by the gas flow indicator device disclosed in our International Patent Application No.: PCT/AU2013/000884, published under WO 2014/026221 A1 on 20 February 2014, and its counterparts in Australia (Application No.: 2013302298), Europe (Application No.: 13829326.1), New Zealand (Application No.: 705892) and the United States of America (Application No.: 14/421,039). The gas flow indicator device of those Applications comprises a gas flow chamber including a transparent portion and an opaque portion, an inlet port, an outlet port, and a gas flow signal means movably disposed within the gas flow chamber; wherein, when there is no gas flow or less than a predetermined gas flow rate, the gas flow signal means is disposed at least substantially within one of the transparent portion and the opaque portion; and wherein, when there is gas flow and the predetermined gas flow rate has been achieved or is being maintained, the gas flow signal means is moved to be disposed at least substantially within the other of the transparent portion and the opaque portion. Typically, the gas flow signal means is biased to a rest position substantially within the one of the transparent and opaque portions and includes a bellows device or a piston device.

While the gas flow indicator device, of our earlier patent Applications identified above, works effectively and is reliable, it is relatively complex and requires careful attention to detail in its manufacture. The present invention is concerned with providing an improved form of gas flow indicator device.

### DISCLOSURE OF THE INVENTION

According to the present invention, there is provided a gas flow indicator device including an elongate housing that has a peripheral sidewall that extends between first and second opposite ends and that defines a gas flow chamber through which gas is able to flow from an inlet port at the first end to an outlet port at the second end. Within the chamber, the device has a gas flow indicator member that extends from adjacent to the first end of the housing over part of the length of the chamber towards the second end. The indicator member is of annular form such that gas flowing through the chamber from the inlet port to the outlet port enters the chamber along a bore that extends through the indicator member. The indicator member is spaced from an inner surface of the sidewall of the housing to define, with that inner surface, an annular space that forms part of the chamber. The device further includes a concealment member that is movable from the first end of the housing in response to a sufficient pressure generated by gas flow from the inlet port to the outlet port, against the action of a biasing member acting to bias the concealment member to the first end. The concealment member has an annular peripheral skirt that, under the bias of the biasing member on the concealment member, is receivable into the annular space, such that one of opposite ends of the skirt is able to seal against an annular surface of the housing at or adjacent to an end wall of the housing at the first end of the housing. The concealment member also includes, at the other of the opposite ends of the skirt, a transverse wall that defines an opening through which the flow of gas from the inlet port to the outlet port is able to pass, with the opening providing resistance to such flow. The arrangement is such that the one of opposite ends of the skirt is able to be held in sealing engagement with the annular surface at or adjacent to the end wall of the housing whereby the indicator member is concealed from view, through a laterally adjacent viewing window portion of the housing when there is no gas flow or a gas flow rate generating less than the sufficient pressure but such that, with increasing gas flow rate the sufficient pressure is achieved and the bias of the biasing member is thereby overcome to enable the concealment member to move towards the second end of the housing and expose the indicator member to view through the viewing window portion of the housing and provide a visual indication indicative of gas flow. The movement of the concealment member to expose the indicator member moves the one of opposite ends of the skirt of the concealment member from the annular surface at or adjacent to the end wall of the housing, and most preferably thereby enables a secondary gas flow to the outlet that passes around the one of opposite ends of the skirt between the concealment member and the housing. Substantial or full exposure of the indicator member preferably shows that not only is gas flowing, but also that a desired or minimum flow rate, such as 6 L/min, has been achieved and is being maintained. The arrangement enables such satisfactory operation to be seen, such as with only a quick glance being all that is required to obtain confirmation of the correct or required operation. In effect, the arrangement is such that the device is readily able to function as if in the nature of an "on" or "off' switch, indicating respectively the there is, or there is not, gas being supplied to a patient (preferably at a desired or minimum flow rate, e.g. such as about 6 L/min).

In one convenient arrangement, the concealment member is made of an opaque material through which the indicator member is not able to be seen when the one end of the skirt of the concealment member is held in sealing engagement with the annular surface at or adjacent to the end wall of the housing. When the concealment member is moved to expose the indicator member, the outer surface characteristics of the indicator member are able to be seen by being directly viewed and, as detailed herein, those characteristics preferably are such as to highlight exposure of the indicator member after movement of the concealment member. However, the concealment member need not be opaque in order to make possible its concealment or exposure of the indicator member. Thus, the concealment member may be semi-opaque, translucent or even transparent but of a material of a colour that interacts with the colour of the indicator member in a visually distinctive manner resulting in an observer being able to readily distinguish between a condition in which the actual colour of the indicator member is concealed, or only visible through the concealment member and seen as if of a colour other than its actual colour, and a condition in which the actual colour of the indicator member can be seen by direct viewing made possible by the concealment member being withdrawn or retracted by a sufficient gas flow rate moving the concealment member against the action of the bias member.

The biasing member may act in compression or in tension in biasing the concealment member to the first end of the housing. Thus, the biasing member may be compressed between, such as compressed by, the concealment member and the second end and provide the required bias in seeking to expand. Alternatively, the biasing member may be within the concealment member, and tensioned by being expanded between, such as by being connected in relation to, each of the transverse wall of the concealment member and the first end of the housing so as to provide the bias in seeking to contract. While other arrangements are possible, the biasing member preferably comprises a coil spring in each of those alternatives.

Where the biasing member is a coil spring acting between the concealment member and the second end of the housing, compression of the spring can limit the extent to which the concealment member can move towards the second end of the housing simply as a consequence of the presence of the compressed spring. Where the biasing member is a coil spring within the concealment member, the arrangement may be such that the concealment member is able to move so as to contact the end wall at the second end of the housing, although the arrangement may be such that contact of the concealment member with the second end of the housing is precluded by the spring reaching its elastic limit.

The concealment member preferably is able to move, between a first position in which the indicator member is concealed and a second position in which the indicator member is sufficiently exposed, or fully or substantially fully exposed, over a distance enabling a clear visual indication of gas flow through the device. The arrangement is such that, when so exposed, the indicator member can readily be seen through the viewing window portion of the sidewall of the housing by a person in close proximity, such as beside the bed of a patient being supplied oxygen through the device, and preferably also from a considerable distance such as from five to ten meters, or further, from the device. In order that the indicator member is viewable, the viewing window portion is at least translucent. However, the window portion preferably is sufficiently transparent to facilitate viewing of the indicator member, when exposed. The viewing window portion most preferably is of high transparency. Also, the viewing window portion most preferably extends around the full circumferential extent of the sidewall of the housing adjacent to the first end, although it could comprise two or more regions spaced circumferentially around that sidewall of the housing adjacent to the first end. Particularly where the viewing window portion extends around the full circumferential extent of the sidewall of the housing, it preferably is of a high-transparency plastics or glass material, with the material preferably also having a high level of clarity. The laterally adjacent portion of the housing, particularly where the viewing window portion extends fully around the circumferential extent of the sidewall of the housing, may be of a different material to that of a remaining extent of the length of the housing and, in that case, the material of which the remainder of the housing is made may be transparent, translucent or opaque, as required. However, the housing preferably is of unitary or integral form and made of a single material, preferable a rigid plastics material such as a suitable engineering plastics material. Where the housing is of unitary or integral form, and made of a transparent material, the remainder of the housing may be rendered opaque by provision of an external or internal lining or coating of an opaque material.

The housing may be of circular in cross-sections perpendicular to the direction of spacing between the first and second ends. The inner surface of the sidewall of the housing may be substantially cylindrical and of substantially constant circular transverse cross-section between the first and second ends, while the sidewall also may have an outer surface that is substantially cylindrical and of substantially constant circular cross-section. However, for reasons explained later herein, the outer surface of the sidewall may depart from such cylindrical form, at least along an initial section of its longitudinal extent from adjacent to the first end, with that initial section extending around the gas flow indicator member. In one convenient arrangement, the outer surface of the sidewall, over that initial section of its longitudinal extent, may smoothly increase in diameter to a maximum in a direction away from the first end towards the second end, and thereafter transition to smoothly decrease in diameter substantially to the diameter at the first end, so as to have a convex longitudinal form. The at least one viewing window portion discussed above preferably has such convex longitudinal form, with the portion of that form most preferably being circumferentially continuous around the housing.

At the first end of the housing, the inlet port may be defined by a spigot that projects from the first end of the housing in a direction away from the second end, and that enables connection of a gas supply conduit to the housing. In one arrangement, the spigot may have an extension into the interior of the housing to provide a tubular extension or hub on or around which the gas flow indicator member can be mounted. In that case, the indicator member may have an axial extent that is substantially the same as, or slightly greater than, the length of the hub. The indicator member may be a firm friction fit on the hub so as to secure the indicator member in position and, to assist in attaining a firm fit, the indicator member and the hub may have complementary surfaces by which they are mutually engaged. The surfaces may taper so as to decrease in cross-section towards the outlet end, for example frustoconically. The taper preferably is relatively slight, such as with a half-cone angle of about two to about six degrees. However other arrangements are possible. For example, the indicator member may be bonded or welded onto the hub, without the need for a friction fit. Alternatively, the indicator member may have an end, adjacent to the end wall at the first end of the housing, by which the indicator member is bonded or welded to the end wall. In a further alternative, the indicator member may have, at its end remote from the first end of the housing, a flange that overlaps the corresponding end of the hub, with the flange bonded or welded to that end of the hub. However, in another arrangement, the spigot does not have an extension that projects into the housing, with the indicator member being bonded or welded to the end wall of the housing at the first end, or mechanically engaged with that end wall such as by screw-threaded engagement or a snap fit in or around the inlet port.

The skirt of the concealment member preferably has an outer surface that is spaced from the inner surface of the sidewall of the housing, although relative sliding contact may be provided between those surfaces. Where there is spacing between the surfaces, this most preferably applies throughout the extent of movement of the concealment member longitudinally within the housing. The inner surface of the sidewall of the housing preferably is of substantially uniform circular cross-section. The device may have guide members that maintain the concealment member in a substantially co-axial relationship with the housing. Such guide members may comprise a plurality of circumferentially spaced projections that stand proud, or project inwardly, of the inner surface of the housing so as to be contactable with the outer surface of the skirt of the concealment member. In one convenient form, the guide members are provided by a plurality of longitudinal ribs on the inner surface of the sidewall of the housing, with three or four such ribs usually being sufficient. In an alternative arrangement, the plurality of guide members may stand proud, or project outwardly, of the outer surface of the skirt of the concealment member. In either case, the outer surface of the skirt of the concealment member may be of substantially uniform circular cross-section, although it preferably has a slight taper so as to decrease in cross-section towards the other end at which the transverse wall of the concealment member is provided, such as a taper providing a half-cone angle of from about two to about six degrees. The guide members not only centralise the concealment member, but also preferably obviate noise or vibrations due the concealment member rattling or oscillating laterally within the housing.

The inner surface of the skirt of the concealment member, when the indicator member is concealed, preferably is spaced from the outer peripheral surface of the indicator member. This avoids contact between the skirt of the concealment member and the indicator member, and thereby obviates friction that could resist movement of the concealment member relative to the indicator member. The skirt of the concealment member and the indicator member each may be of uniform wall thickness along its respective axial extent, while they may be of similar or substantially the same wall thickness.

At the second end, the housing may have an end wall that defines the outlet port. The end wall preferably is separable from the sidewall of the housing. In one convenient arrangement, the end wall comprises an annular peripheral rim that is engaged with the sidewall of the housing around the second end, a central hub portion and a plurality of circumferentially spaced connectors that extend between the rim and the hub.

According to a further aspect of the present invention, there is provided a gas delivery device, gas delivery system or gas supply conduit including the gas flow indicator device of any one of the preceding paragraphs.

These and other essential or preferred features of the present invention will be apparent from the description that now follows.

### BRIEF DESCRIPTION OF THE DRAWINGS

In order that the invention may be more clearly understood and put into practical effect there shall now be described in detail preferred constructions of a gas flow indicator device for gas delivery devices or systems and gas supply conduits in accordance with the invention. The ensuing description is given by way of non-limitative examples only and is with reference to the accompanying drawings, wherein:
Figure 1 is a schematic representation of gas flow indicator device according to the invention as incorporated into a face mask installed on a patient to receive a controlled gas supply;
Figure 2 shows a perspective view of a first preferred embodiment of a gas flow indicator device according to the invention, shown in an operating condition;
Figure 3 is an exploded view of the device of Figure 2, shown in a non-operating condition in relation to a face mask;
Figure 4 is perspective view from one end of the device of Figure 2, this time shown in a non-operating condition;
Figure 5 is similar to Figure 4 but taken from the other end;
Figure 6 is a side elevation showing the device of Figure 2 in the non-operating condition applicable to each of Figures 4 and 5;
Figure 7 corresponds to Figure 6, but shows the device in its operating condition;
Figures 8 and 9 correspond to views Figures 4 and 5, respectively, but in exploded views on an enlarged scale;
Figure 10 is a plan view from one end of the device of Figure 2;
Figure 11 is a sectional view of the device of Figure 2, taken on line X-X of Figure 10;
Figure 12 corresponds to Figure 10, but is taken from the opposite end;
Figure 13 is a schematic representation of the device of Figure 2, illustrated in operation in accordance with the condition shown in Figure 7;
Figures 14 and 15 correspond to Figures 6 and 7, respectively, but with one component sectioned to better illustrate internal components;
Figures 16 to 18 show different preferred forms for one component of the device of Figure 2;
Figure 19 shows the device of Figure 2, in a view corresponding to Figure 6, as incorporated into a conduit flow-line;
Figure 20 corresponds to Figure 19, but shows the device and flow-line in longitudinal section;
Figures 21 to 24 illustrate gas flow indicator device according to a second embodiment of the invention, shown in views corresponding to Figures 6, 11, 13 and 10, with Figures 22 and 23 taken on line Y-Y of Figure 24;
Figures 25 to 28 show an end elevation, a side elevation, a perspective view and a section view taken on line Z-Z of Figure 26, respectively of a component suitable in the device of Figures 2 to 20, or the device of Figures 21 to 24;
Figures 29 to 36 provide respective sectional views illustrating alternatives for securing together two components for the device of the illustrated first or second embodiments of the invention shown in Figures 2 to 20 and Figures 21 to 24 respectively;
Figure 37 is similar to Figure 15, but highlights an encircled region of the device;
Figure 38 illustrates performance with the encircled region of Figure 37; and,
Figures 39 to 42 illustrate different detail for components of the device of Figure 2, but also applicable to the device of Figures 21 to 24.

### MODES FOR CARRYING OUT THE INVENTION

In the following detailed description of the invention, reference is made to the drawings in which like reference numerals refer to like elements throughout, and which are intended to show by way of illustration specific embodiments in which the invention may be practiced. It is to be understood that other embodiments may be utilised and that procedural and/or structural changes may be made without departing from the scope of the invention.

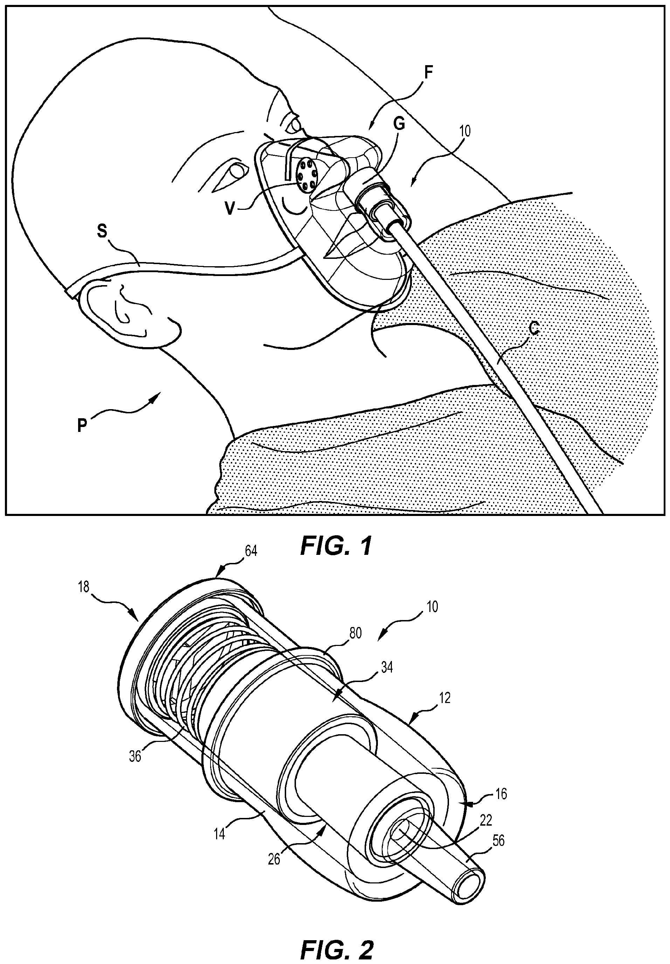

Figure 1 schematically illustrates a patient P undergoing a procedure in which the patient P is receiving a controlled supply of required gas through a device 10 according to the invention. The device 10 is fitted into a face mask F held by strap S over the nose and mouth of the patient P and the required gas is passed to the device 10 from a source of supply (not shown) via a conduit C.

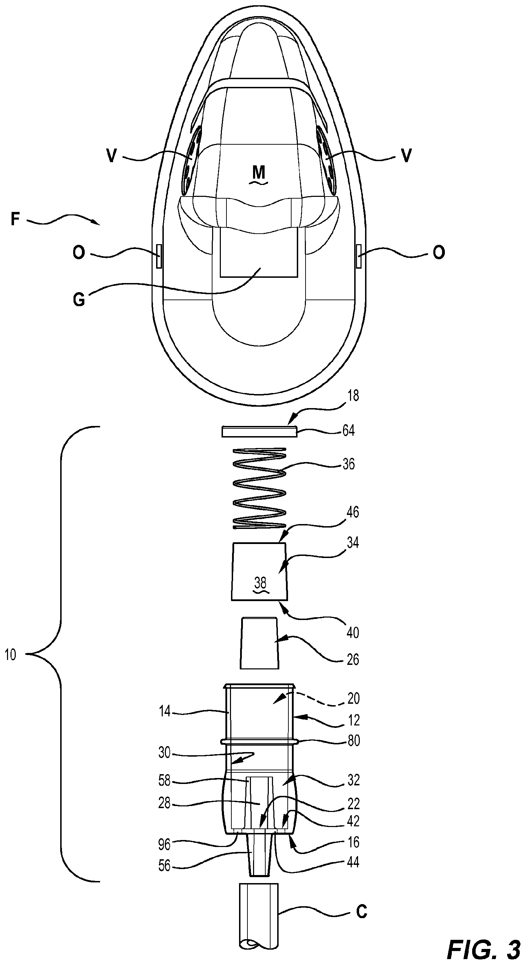

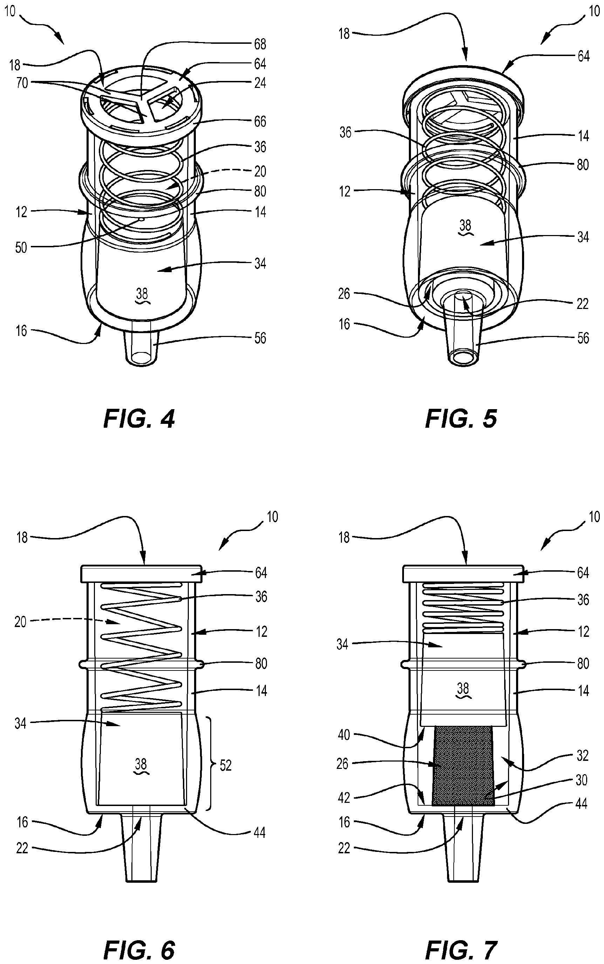

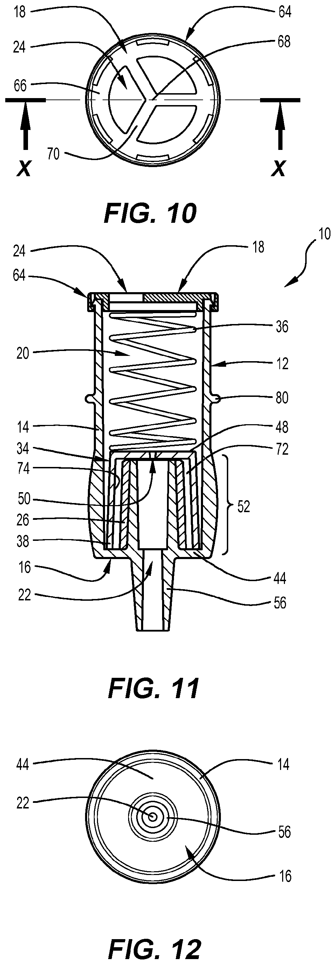

The device 10 comprises a gas flow indicator device that, as shown in Figures 2 to 15, is of elongate form. Device 10 includes an elongate housing 12 that, as shown in Figure 3, has a peripheral sidewall 14 that extends between first and second opposite ends comprising an inlet end 16 and an outlet end 18, respectively. The housing 12 defines a gas flow chamber 20 through which gas is able to flow from an inlet port 22 at the inlet end 16 to an outlet port 24 (see, for example, Figure 4) at the outlet end 18. Within the chamber 20 the device 10 has a gas flow indicator member 26 that extends from adjacent to the inlet end 16 of the housing 12 over part of the length of the chamber 20 towards the outlet end 18, typically less than half the length of the chamber 20, such as from 35 to 45% of the length. The indicator member 26 is of annular form such that gas flowing through the chamber 20 from the inlet port 22 to the outlet port 24 enters the chamber 20 along a bore 28 that extends through the indicator member 26. The indicator member 26 is spaced from an inner surface 30 (see, for example, Figure 7) of the sidewall 14 of the housing 12 to define, with that inner surface 30, an annular space 32 (again, see for example, Figure 7) that forms part of the chamber 20.

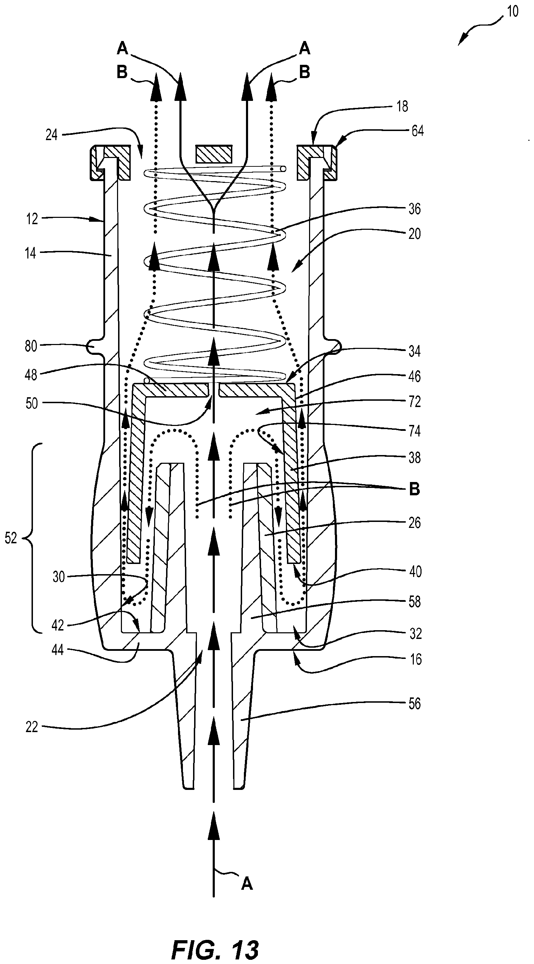

The device 10 has a concealment member 34 that is movable from the inlet end 16 of the housing 12 in response to a sufficient pressure generated by gas flow from the inlet port 22 to the outlet port 24. However, such movement of concealment member 34 is against the action of a biasing member 36 acting to bias the concealment member 34 to the first end 16 of housing 12. The concealment member 34 has an annular peripheral skirt 38 that is receivable into the annular space 32, such that one end 40 of the skirt 38 is able to seal against an annular abutment surface 42 defined by housing 12 at or adjacent to an end wall 44 of the housing 12 at the inlet end 16 of the housing 12. The concealment member 34 also includes, at the other end 46 of the skirt 38, a transverse wall 48 that defines an opening 50 through which the flow of gas from the inlet port 22 to the outlet port 24 is able to pass, with the opening 50 providing resistance to such flow. The arrangement is such that the end 40 of the skirt 38 is able to be held in sealing engagement with the annular abutment surface 42 of the housing 12 whereby the indicator member 26 is concealed from view, through a laterally adjacent viewing window portion 52 of the housing 12 that is at least translucent until, with increasing gas flow rate, the sufficient pressure is achieved and the bias of the biasing member 36 is thereby overcome to enable the concealment member 34 to move towards the outlet end 18 of the housing 12 and expose the indicator member 26 to view through the viewing window portion 52 of the housing 12 and provide a visual indication indicative of gas flow. As already outlined above, it is preferred that full exposure of the indicator member 26 shows that not only is gas flowing, but also that a desired or minimum predetermined flow rate, such as a flow rate of about 6 L/min, has been achieved and is being maintained. The movement of the concealment member 34 to expose the indicator member 26 moves the end 40 of the skirt 38 of the concealment member 34 from the annular abutment surface 42 of the housing 12, and most preferably thereby enables a secondary gas flow to the outlet port 24 that passes around the end 40 of the skirt 38 between the concealment member 34 and the housing 12 (as will be described in further detail below, with reference to Figure 13).

In the arrangement of Figures 2 to 15, the biasing member 36 is a coil spring that acts in compression in acting to bias the concealment member 34 to the inlet end 16 of the housing 12. Although the use of coil spring is described, and shown in the drawings, it will be appreciated that any suitable form of biasing member 36 may be utilised in accordance with the invention. As is shown in Figures 2 to 20, the preferred coil spring biasing member 36 may be compressed by and between the concealment member 34 and the outlet end 18 of the housing 12 so as to provide the required bias in seeking to expand. The concealment member 34 is able to move against the bias from the position shown Figures 4 to 6, in which the indicator member 26 is concealed, to the position shown in Figures 2 and 7 in which the indicator member 26 is sufficiently exposed, over a distance enabling a clear visual indication of gas flow through viewing window portion 52 of the housing 12 of the device 10. The arrangement is such that, when exposed, the indicator member 26 can readily be seen through the laterally adjacent viewing window portion 52 of the sidewall 14 of the housing 12 by a person in close proximity, such as beside the bed of patient P of Figure 1, and preferably also from a considerable distance such as from five to ten meters, or further, from the device 10 provided for patient P. Thus, the arrangement of device 10 is such that its operating condition can be readily determined, such as with only a quick glance being all that is required to obtain confirmation of the operation condition. The arrangement is such that device 10 is readily able to function, in effect, as if in the nature of an "on" or "off' switch, indicating respectively the there is, or there is not, gas being supplied to a patient P (preferably at a desired or minimum flow rate, e.g. such as about 6 L/min).

While the laterally adjacent viewing window portion 52 is at least translucent, it preferably is transparent to facilitate viewing of the indicator member 26, when exposed. The viewing window portion 52 most preferably is of high transparency, and most preferably extends around the full circumferential extent of the sidewall 14 of the housing 12 adjacent to the first end 16, as shown in each of Figures 2 to 15. However, such window portion could comprise two or more regions spaced circumferentially around sidewall 14. Particularly where the viewing window portion 52 extends around the full circumferential extent of the sidewall 14, it preferably is of a high-transparency plastics or glass material, with the material preferably also having a high level of clarity. The laterally adjacent viewing window portion 52, particularly where it extends fully around the circumferential extent of the sidewall of the housing 12, may be of a different material to that of a remaining extent of the length of the housing 12 and, in that case the material of which the remainder of the housing 12 is made may be transparent, translucent or opaque, as required. However, the housing 12 preferably is of unitary or integral form and made of a single material, preferable a rigid plastics material such as a suitable engineering plastics material.

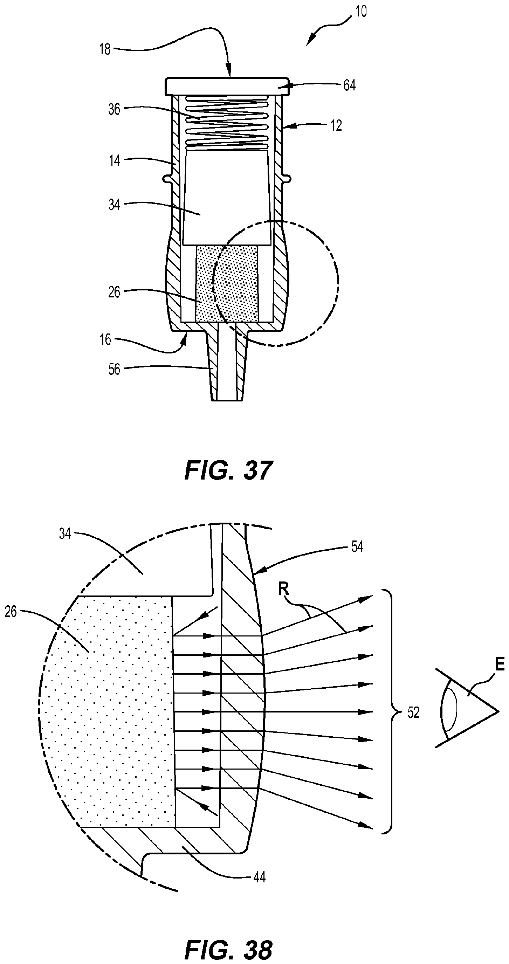

The housing 12 is of circular in cross-sections perpendicular to the direction of spacing between the inlet and outlet ends 16 and 18, of device 10. The inner surface 30 of the sidewall 14 of the housing 12 may be substantially cylindrical and of substantially constant circular transverse cross-section between the first and second ends 16 and 18, while the sidewall 14 also may have an outer surface that is substantially cylindrical and of substantially constant circular cross-section. However, as shown in each of Figures 2 to 15, for reasons explained later herein with reference to Figures 37 and 38, the outer surface 54 of the sidewall 14 departs from such cylindrical form along an initial section of its longitudinal extent from adjacent to the first end 16, that substantially corresponds to the viewing window portion 52. Over that initial section, the outer surface 54 of the sidewall 14 smoothly increases in diameter to a maximum in a direction away from the inlet end 16 towards the outlet end 18, and thereafter smoothly decreases in diameter substantially to the diameter at the first end 16, so as to have a convex longitudinal form. The convex longitudinal form most preferably is circumferentially continuous around the housing 12.

The inlet port 22 is defined by a spigot 56 that projects from the inlet end of the housing 12 in a direction away from the outlet end 18, with the spigot 56 enabling connection of device 10 to a gas supply conduit C. The spigot 56 has an extension that projects into the interior of the housing 12 to provide a tubular hub 58 on or around which the gas flow indicator member 26 is securely mounted. The indicator member 26 has an axial extent that is substantially the same as, or slightly greater than, the length of the hub 58. The indicator member 26 may be a firm friction fit on the hub 58 so as to secure the indicator member 26 in position and, to assist in attaining a firm fit, the indicator member 26 and the hub 58 may have complementary surfaces by which they are mutually engaged. The surfaces may taper so as to decrease in cross-section towards the outlet end 18, for example frustoconically, such as with a half-cone angle of about two to about six degrees. Alternatively, the indicator member 26 may be bonded or welded onto the hub 58, without the need for a friction fit. In a further alternative, the indicator member 26 may have an end adjacent to the end wall 44 at the inlet end 16 of the housing 12, by which the indicator member 26 is bonded or welded to the end wall 44. In another alternative, the indicator member 26 may have, at its end remote from the inlet end 16 of the housing 12, a flange that overlaps the corresponding end of the hub 58, with the flange bonded or welded to that end of the hub 58. However, in another arrangement (not shown), the spigot 56 does not have an extension that projects into the housing, in which case the indicator member 26 may be bonded or welded to the end wall 44 of the housing 12, or mechanically engaged with that end wall 44 such a by screw-threaded engagement or a snap-fit in or around the inlet port 22.

The skirt 38 of the concealment member 34 has an outer surface 60 that is spaced from the inner surface 30 of the sidewall 14 of the housing 12, although relative sliding contact may be provided between those surfaces. Where there is spacing between the surfaces 30 and 60, this most preferably applies throughout the extent of movement of the concealment member 34 longitudinally within the housing 12. The inner surface 30 of the sidewall 14 of the housing 12 preferably is of substantially uniform circular cross-section. The outer surface 60 of the skirt 38 of the concealment member 34 may be of substantially uniform circular cross-section although, as shown, it preferably has a slight taper so as to decrease in cross-section towards the end at which the transverse wall 48 of the concealment member 34 is provided. The slight taper may be such as to provide a half-cone angle of from about two to about six degrees. The inner surface of the skirt 38 of the concealment member 34, when the indicator member 26 is concealed, is preferably spaced from the outer peripheral surface 62 of the indicator member 26. This avoids contact between the skirt 38 of the concealment member 34 and the indicator member 26, and thereby obviates friction that could resist movement of the concealment member 34 relative to the indicator member 26. The skirt 38 of the concealment member 34 and the indicator member 26 each may be of uniform wall thickness along its respective axial extent, while they may be of similar or substantially the same wall thickness.

At the outlet end 18 of device 10, the housing 12 has an end wall 64 that defines the outlet port 24. The end wall 64 preferably is separable from the sidewall 14 of the housing 12. In the arrangement shown in Figures 2 to 15, the end wall 64 comprises an annular peripheral rim 66 that is engaged with the sidewall 14 of the housing 12 around the outlet end 18, a central hub 68 and a plurality of circumferentially spaced connectors 70 that extend between the rim 66 and the hub 68. The coil spring comprising biasing member 36 is positioned coaxially within housing 12 and with respect to transverse wall 48 of concealment member 34 and end wall 64. A respective end of biasing member 36 may be attached to walls 48 and 64, or the member 36 may simply be held between walls 48 and 64 under compression. The arrangement of end wall 64 is such that it forms an end cap for device 10 that not only provides a means of retaining component parts of device 10 within housing 12, but also acts as a grill that prevents foreign objects from entering into device 10.

In use, required gas such as oxygen or oxygen-enriched air is supplied through device 10. In the arrangement of Figure 1, for example, the gas is supplied through conduit C so as to flow through spigot 56 and then through inlet port 22 and bore 28. From bore 28, the gas flows into a sub-chamber 72 defined within concealment member 34, indicator member 26 and end wall 44 of housing 12, before flowing through opening 50 of transverse wall 48, of concealment member 34, to pass into chamber 20, before exiting through outlet port 22 into facemask F. The gas received into mask F then can be inhaled by patient P, and is supplied at a rate appropriate for the patient's P needs. Balancing the pressure at which the gas is supplied to conduit C, and the resistance to the flow of gas through conduit C and through device 10, enables compliance with the patient's P needs. Within device 10, the resistance to flow is determined by the size of opening 50 relative to the area of transverse wall 48, of concealment member 34, and the compressibility of the spring comprising biasing means 36. Opening 50 is such as to resist gas flow from sub-chamber 72 to chamber 20, causing a generation of gas pressure against transverse wall 48 that, supplemented by pressure against the tapered inner surface 74 of skirt 38, acts to oppose the bias of biasing member 36 and tends to move concealment member 34 away from the inlet end 16 of housing 12, towards the outlet end 18. The resistance to gas flow provided by opening 50 is designed to result in the pressure in sub-chamber 72 increasing to a level at which such movement of concealment member 34 occurs, with consequential breaking of the sealing engagement between the end 40 of skirt 38 of concealment member 34 and the annular abutment surface 42 defined by housing 12. The gas flow through opening 50 is somewhat centralised in a gas stream along an axial line through device 10, as depicted in Figure 13 by solid-line arrows A-A. However, with that break in sealing engagement between end 40 of skirt 38 and abutment surface 42, a second gas stream commences, with this being radial, outwardly between end 40 and abutment surface 42, and then as an annular curtain of gas passing to outlet port 24 between the inner surface 30 of sidewall 14 of housing 12 and the outer surface 60 of skirt 38 of concealment member 34. In Figure 13, the dotted-line arrows B-B depict the second, annular curtain gas stream.

As illustrated in Figure 13, the pressure of gas within the sub-chamber 72 of the concealment member 34, against transverse wall 48, acts against the bias of biasing means 36 and enables initiation of movement of concealment member 34 that permits generation of the second gas stream depicted by arrows B-B. This results in a reduction of pressure within concealment member 34, although the reduced pressure against wall 48 is supplemented by gas pressure against the annular area of end 40 of skirt 38, as well as a reduced pressure prevailing over the tapered outer surface 60 of skirt 38 relative to the pressure over the tapered inner surface 74 of skirt 38. The overall balance achieved with application of a gas supply pressure within a typical range is such that the concealment member 34 is moved against the biasing member 36 a distance exposing a sufficient axial extent of indicator member 26 whereby indicator member 26 is visible through the laterally adjacent viewing window portion 52 of housing 12. The arrangement is such that concealment member 34 typically is moved beyond the position shown in Figure 13, such as to a position able to be seen in each of Figures 2, 7 and 15, so that the external surface 62 of indicator member 26 is exposed and able to be viewed through the laterally adjacent window portion 52 of housing 12. For this, the spacing within chamber 20 between the outlet end 18 of housing 12 and the nearer end of indicator member 26 is able to accommodate concealment member 34 and the compressed coils of the spring comprising biasing member 36. However, with termination of or an interruption to gas flow through device 10, the bias provided by biasing member 36 acts to return the concealment member to its position in which it conceals indicator member 26 from view.

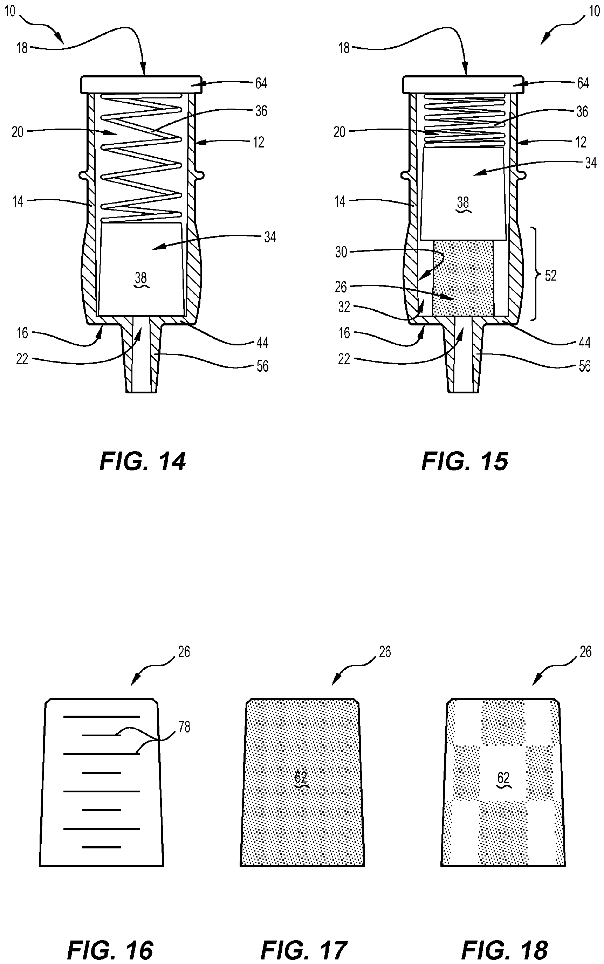

The indicator member 26 preferably is made of or coated with a material that maximises its visibility. Thus, the material may have a strong, vibrant colour, or it may be iridescent, fluorescent or highly reflective, so as to draw the eye E of a person charged with observing the patient P from time to time to ensure a required supply of gas is being maintained. In addition to such materials and effects, the outer surface 62 of indicator member 26 may be marked, textured or patterned as shown in Figures 16 to 18. Thus, Figure 16 shows indicator member 26 as provided graduated markings 78 facilitating fine-tuning and observance of maintenance of the flow of gas through device 10, such as by appropriate adjustment of a source of gas supply. Figure 17 shows an indicator member 26 having an outer surface 62 that is textured to maximize its exposure being observed, even in low lighting conditions. Figure 18 shows an indicator member 26 that is patterned in a checkerboard to provide a plurality of areas differing in colour, texture or both colour and texture.

Reverting to Figure 3, which shows an example of a face mask F with which the device 10 is usable. The mask F has a main body M, a gas inlet G, ventilation apertures V and, around the edge of body M, openings O by which, as shown in Figure 1, the mask F can be retained in position on the face of a patient P by straps S. The gas inlet G is of tubular form and the outlet end 18 of device 10 is a neat sliding fit in inlet G. The device 10 has a peripheral bead 80 around the exterior of housing 12 that ensures the outlet end 18 of device 10 is not inserted too far into inlet G of face mask F.

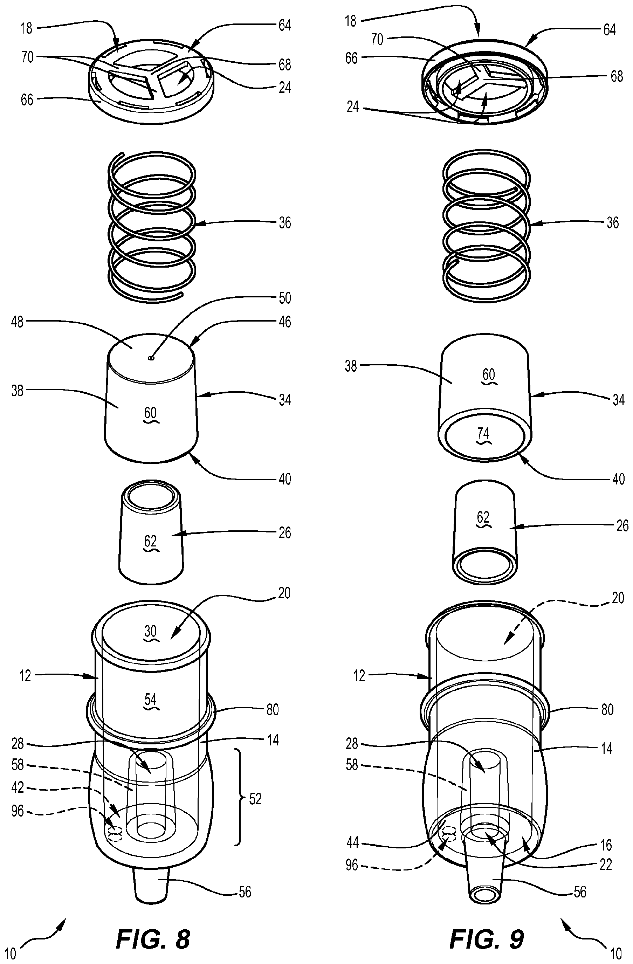

In the form of device 10 depicted in Figures 8, 9, 11 and 13, ease of assembly of device 10 readily can be appreciated. The indicator member 26 first is inserted into the outlet end 18 of housing 12 and fitted onto and secured in relation to the hub 58 provided as an extension of spigot 56. The concealment member 34 then is positioned over indicator member 26 to position the inlet end 40 of skirt 38 of member 34 against the abutment surface 42. The coil spring to comprise biasing member 36 then is positioned within the outlet end 18 of housing 12 and compressed down onto transverse wall 48 of concealment member 34 by application of end wall 64 so as to securely engage the rim 66 of wall 64 onto the outlet end 18 of housing 12.

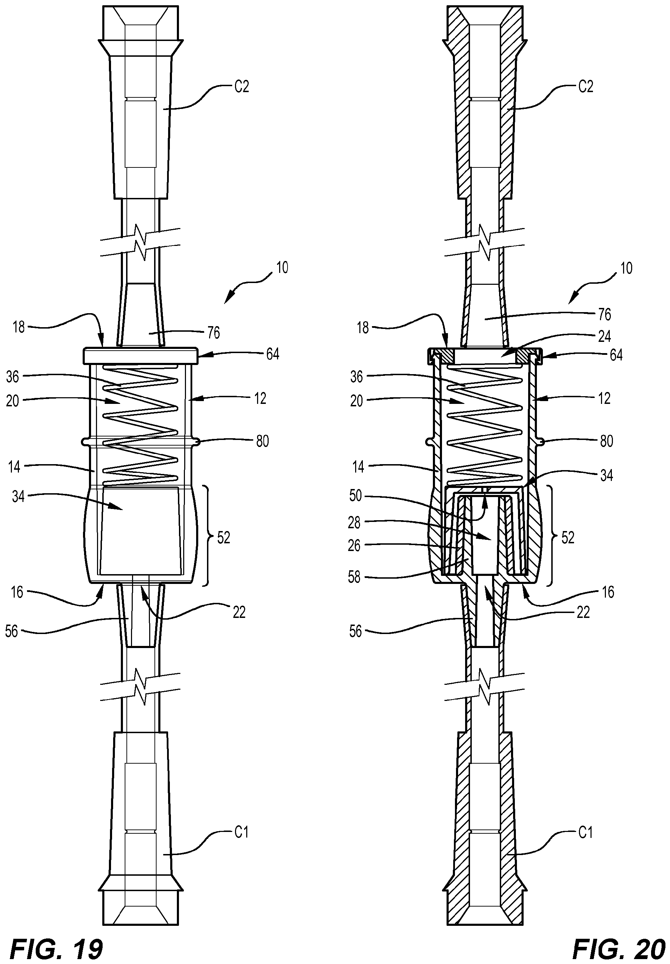

Figures 19 and 20 show device 10 in a different arrangement to that of Figure 1. In Figures 19 and 20, the device 10 is installed in a conduit line that has an inlet conduit length C1 connected onto spigot 56 in the manner shown for conduit C of Figure 1. However, member 10 is provided with an outlet end wall 64 that has an integral spigot 76 that projects axially away from housing 12. The spigot 76 has a second conduit length C2 connected, with gas passing through device 10 being received into conduit length C2 by which the gas is passed to a required location. The required location maybe, for example, a face mask, bag valve mask or endotracheal tube, etc.

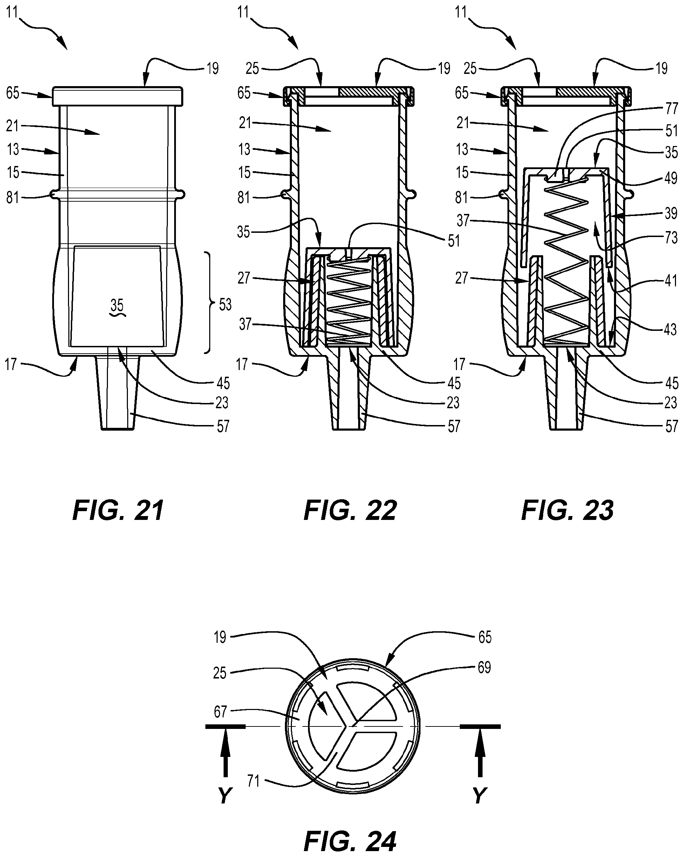

Figures 21 to 24 illustrate an alternative form of device 11 according to the invention. The structure of device 11 generally will be understood from the description of device 10 of the preceding Figures, and corresponding components have the same reference numeral, plus 1. Description of Figures 21 to 24 therefore will be limited to principle matters of difference between device 10 and device 11. The principle feature of difference resides in the device 11 having a biasing member 37 preferably comprising a coil spring that is located within the concealment member 35. The biasing member 37 is in tension so that the inlet end 41 of skirt 39 of the concealment member 35 is pulled into sealing engagement with an annular abutment surface 43 defined by housing 13 at or adjacent to an end wall 45 of the housing 13 at the inlet end 17 of the housing 13. The biasing member 37 has one of opposite ends engaged with end wall 45 of housing 13, around the inlet port 23. The other of the opposite ends of the biasing member 37 is secured to an annular boss 77 that is provided on the transverse wall 49 of concealment member 35, within sub-chamber 73, and that defines the opening 51 in transverse wall 49. Thus, as can be appreciated from a comparison of Figures 22 and 23, the movement of concealment member 35 to expose the indicator member 27 increases tension in the biasing means 37. Termination or interruption of gas flow through device 11 thus results the bias provided by member 37 returning concealment member 35 to its position in which it conceals indicator member 27 and prevents member 27 from being viewed through window portion 53 of housing 13.

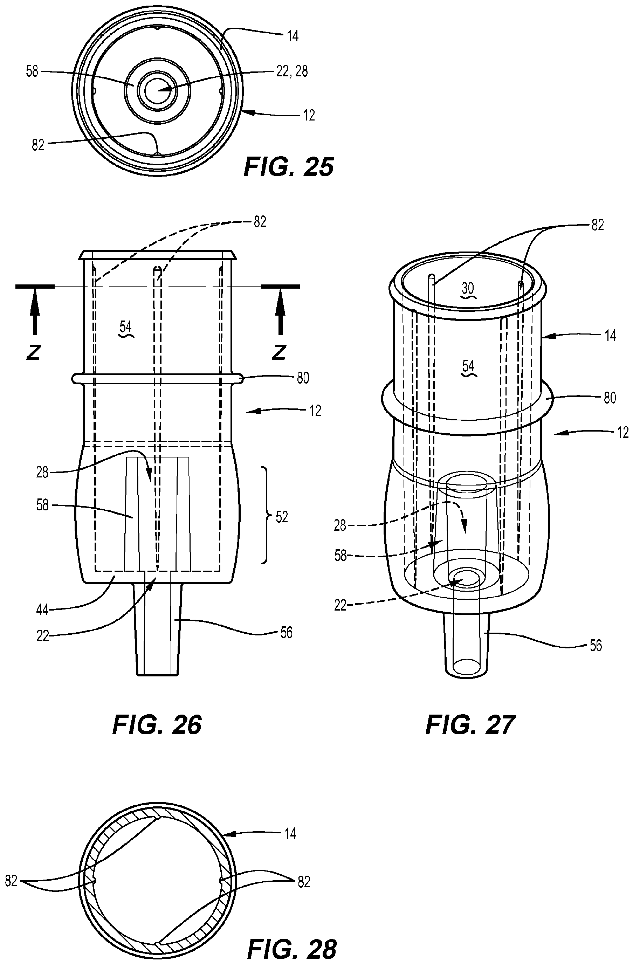

Figures 25 to 28 show a housing 12 suitable for the device 10 shown in Figures 2 to 20, but with a modification that also can be adopted in the housing 13 of the device 11 of Figures 21 to 24. The modification is in the provision, along the inner surface 30 of the sidewall 14 of housing 12, of a number of longitudinally extending, circumferentially spaced ribs 82. In the arrangement shown, there are four ribs 82, although there may only be three (or less) or there may be more than four ribs 82. The ribs 82 preferably are uniformly spaced from each other around inner surface 30 of housing 12, while the cross section of each rib 82 is such that it substantially bridges the spacing between the inner surface 30 of housing 12 and the outer surface 60 of skirt 38 of concealment member 34 without impeding movement of the concealment member 34 with or against the bias of the biasing member 36. Despite this, the ribs 82 do serve to centralize the concealment member 34 during such movement and when stationary. Ribs 82 also serve to obviate noise or vibrations due to the concealment member 34 rattling or oscillating laterally within housing 12 during use of device 10.

It is to be appreciated that while ribs 82 are illustrated in Figures 25 to 28, other alternative centralizing formations could be provided. For example, inner surface 30 of sidewall 14 of housing 12 could have formed thereon a plurality of small protrusions that are spaced over, both along and around, the inner surface 30. In a further alternative, the centralizing and/or noise reduction formations, such as ribs or protrusions could be provided over the outer surface 60 of skirt 38 of concealment member 34, rather than over inner surface 30 of the sidewall 14 of housing 12.

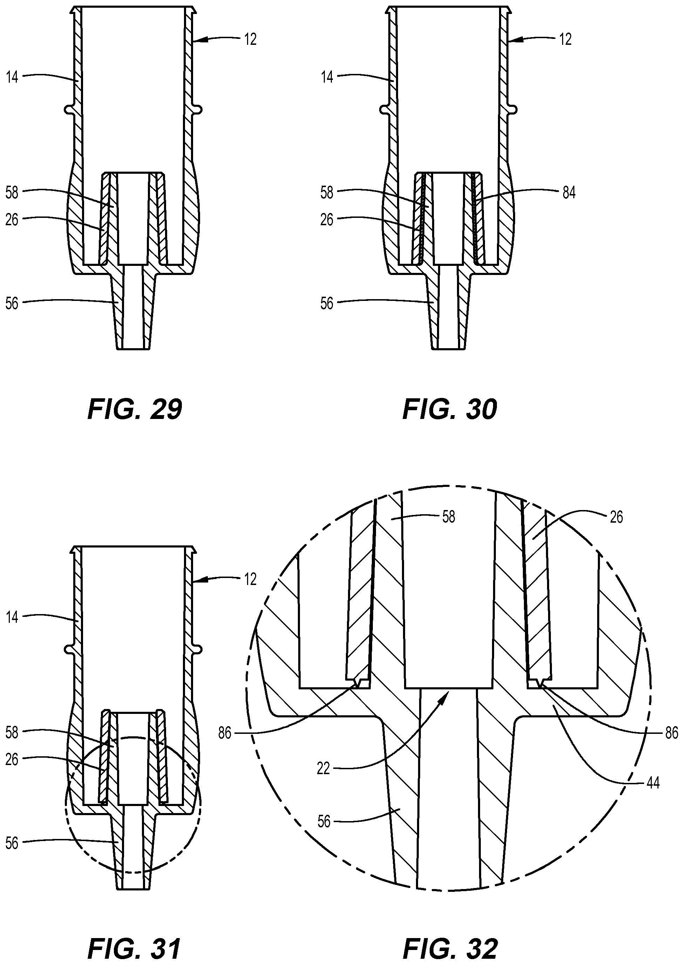

Figures 29 to 32 illustrate alternative construction detail by which indicator member 26 may be secured concentrically on the hub 58 comprising a continuation of spigot 56 of device 10 of Figures 2 to 20. The same alternatives apply to the securement of the indicator member 27 on the hub 59 in the device 11 of Figures 21 to 24. In Figure 29, the indicator member 26 is a neat fit on hub 58, such as to provide a strong friction fit or with securement enhanced by a film providing adhesive bonding. Figure 30 shows an arrangement in which concealment member 26 is spaced slightly from hub 58 but is secured by an adhesive bonding layer 84 between the opposed surfaces of member 26 and hub 58. In the alternative of Figure 31, more clearly illustrated by the enlarged detail of Figure 32, a fusible fin 86 is provided around the end of indicator member 26 opposed to end wall 44 of housing 12 and, when the fin 86 is heated by any suitable arrangement, the fin 86 melts to bond that end of member 26 to wall 44.

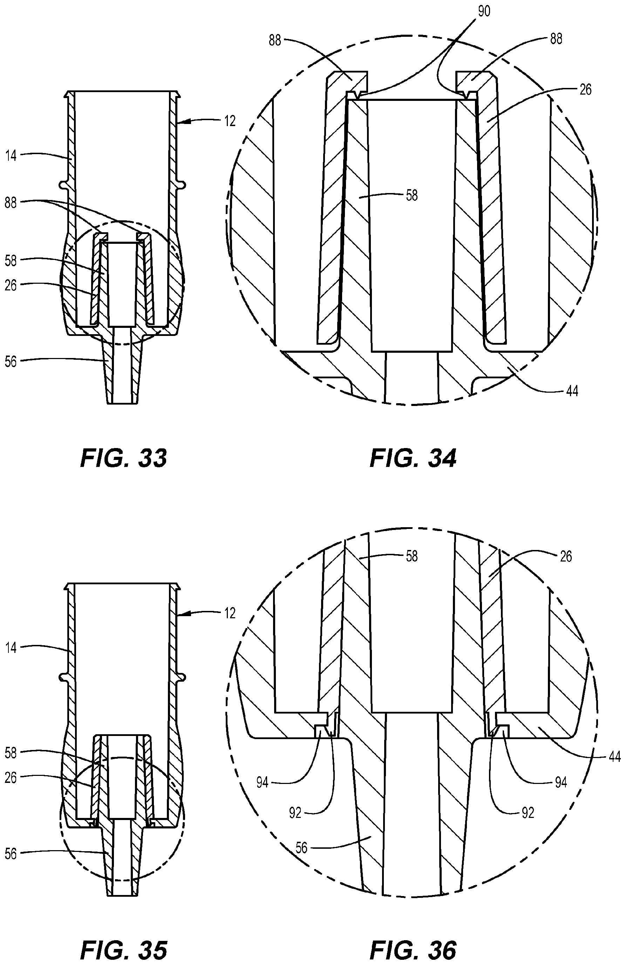

Further alternatives for securing indicator member 26 on hub 58 of device 10, with similar possible for securing member 27 on hub 59 of device 11, are shown in Figures 33 and 35, with Figures 34 and 36 providing respective enlarge detail. In the arrangement of Figures 33 and 34, the indicator member 26 is shown as having an in-turned flange 88 around the end of member 26 remote from end wall 44 of housing 12. The flange 88 overlaps the adjacent end of hub 58, with a fin 90 on flange 88 bearing against that adjacent end of hub 58. Heating of the fin 90 by any suitable arrangement enables the fin 90 to melt, with indicator member 26 then able to move more firmly onto hub 58 and be secured by flange 88 being fusion bonded to the adjacent end of hub 58. In the case of Figures 35 and 36, there is provided a mechanical securement of indicator member 26 onto hub 58. For this, the end of indicator member 26 opposed to end wall 44 of housing 12 has an array of circumferentially spaced, axially projecting hooked fingers 92. As indicator member 26 is moved onto hub 58, each finger 92 is forced to elastically flex so as to enter a respective aligned opening 94 through end wall 44 of housing 12. When indicator member 26 is fully received onto hub 58, each finger 92 is able to elastically recover, to locate its out-turned end against the outer surface of end wall 44 and thereby lock indicator member 26 on hub 58.

Figure 37 and, in particular, the enlarged detail of Figure 38, elaborates on the description above in relation to the viewing window portion 52 of the housing 12 of device 10 shown in Figures 2 to 20, with the same being applicable to the corresponding features of device 11 of Figures 21 to 24. As can be recognized from the schematic view of Figure 38, in particular, the longitudinally arcuate form of the outer surface 54 of housing 12 within the window portion 52, enables window portion 52 to function as a magnifying lens. The effect is such that light rays R shown as issuing out from indicator member 26 diverge out from window portion 52. While the eye E of an observer is depicted as close to window portion 52, the lens effect is such as to permit viewing of indicator member 26 from a considerable distance, as indicator member 26 is seen as enlarged relative to its actual size and also to be viewed over a wider viewing angle. These factors, combined with the colour and other highlighting features of indicator member 26, facilitate indicator member 26 being readily viewed when it is exposed by movement of concealment member 34, as a consequence of a desired or required flow of gas through the device 10.

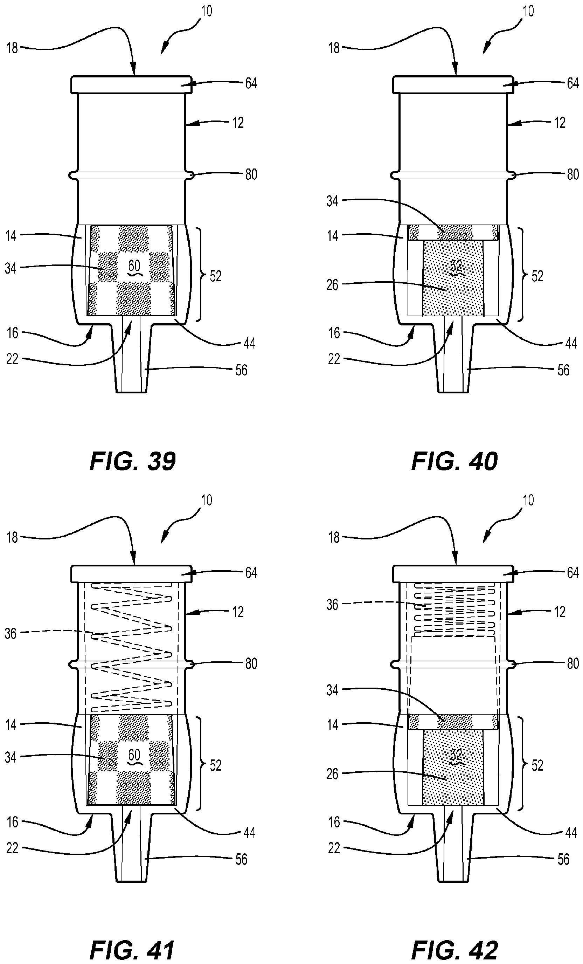

Figures 39 to 42 illustrate further variants possible to assist with recognition of the operating condition of device 10 of Figures 2 to 20, with the same being applicable to device 11 of Figures 21 to 24. Figures 39 and 41 show the same condition for device 10, with the indicator member (not visible) concealed by the concealment member 34, although Figure 41 shows internal structure consistent with this. Figures 40 and 42 also are the same condition for device 10, but with the indicator member 26 exposed as a consequence of movement of the concealment member 34 in response to a required gas flow, although Figure 42 shows internal structure consistent with this. The variant depicted in Figures 39 to 42 is the provision of patterning, colouring and texture differences adopted for the external surface 60 of the skirt 38 of concealment member 34, relative to the outer surface 62 of the indicator member 26. The contrast between surfaces 60 and 62 is such that a viewer is even more readily to distinguish the surface 60, seen through the window portion 52 when concealment member 34 is concealing the indicator member 26, from the surface 62 seen when indicator member 26 is exposed by movement of concealment member 34 as a consequence of a required flow of gas through device 10. Thus, checking that gas is flowing, as required can be even more reliably and readily confirmed. Also, as is evident from Figures 39 to 42, the length of the housing 12 between the transparent window portion 52 and the second end 18 may be opaque (or coated with an opaque material or coating, etc.) and of any suitable colour, thereby serving to maximise the visual contrast between the respective conditions in which the indicator member 26 is concealed or exposed. Also, where device 10 is to be one of a series as discussed later herein, that opaque length of the housing 12 may carry indicia that distinguishes that device 10 from others of the series.

At, or adjacent to the inlet end 16 or 17 of the respective devices 10 or 11, there may be at least one opening enabling ambient air to be drawn through the respective chamber 20 or 21, to mix with gas such as oxygen or oxygen-enriched air being supplied to a patient. The opening may be in the end wall 44 or 45, such as shown by hole 96 in device 10 of Figures 3, 8 and 9. Alternatively the, or each, opening may be in the side wall 14 or 15 of respective device 10 or 11, closely adjacent to the end wall 44 or 45. Where the opening or openings is/are in end wall 44 or 45, its/their position is such that it/they is/are fully covered by the nearer end 40 or 41 of skirt 38 or 39 of the concealment member 34 or 35 when that end 40 or 41 of the skirt 38 or 29 is in sealing engagement with the end wall 44 or 45. In any event, ambient air is precluded from being drawn into the chamber 20 or 21, through the opening or openings, until the seal between end 40 or 41 of skirt 38 or 39, of concealment member 34 or 35, is broken by the flow rate of the supply gas increasing to generate the sufficient pressure to cause the concealment member 34 or 35 to move against the action of the biasing member 36 or 37, to enable the secondary flow of the supplied gas around the exterior of the concealment member 34 or 35. It is the secondary flow, in passing across the at least one opening that draws in the ambient air, by creating a Venturi effect.

As will be appreciated, the gas flow indicator device of the invention enables enhanced ease of observation of a condition, namely exposure of the indicator member, confirming required supply of gas to a patient. With full exposure of the indicator member preferably also confirming that a desired or minimum gas flow rate, such as 6 L/min, has been achieved and is being maintained. The device has components that enable low cost production, readily assembled and provide reliable operation. Also, the device is such that it can be provided in a number of alternative forms each suited to providing a respective required gas flow rate. Thus, for a given standardised biasing member, it is possible to vary performance by selecting from a series of devices that differ in the size of the opening of respective concealment members and, hence, the gas flow rate required to move the concealment member to expose the respective indicator member. Alternatively, a series of devices may have a standardised such opening in the respective concealment members, but differ in the strength of the biasing action of the respective biasing members. Thus, in each case, a respective gas flow rate is required for each device of the series in order to overcome the resistance to flow provided by the respective opening of the concealment member in order for the concealment member to move against the action of the biasing member. The different devices of such series can be readily differentiated by the colour or indicia provided on the outer surface of the skirt of each concealment member, or elsewhere on the device, such as on the housing. Of course, the device also may be provided in a single standardised form, with the gas flow rate at any time within a suitable range of flow rates being apparent from the extent of exposure of the indicator member.

All components of the device of the invention are able to be of nonferrous materials, indeed none of the components need be metallic, making the device suitable for use with a face mask supplying gas to a patient while the patient is undergoing an MRI or other procedure precluding metals. Also, the device can be used in any orientation, in contrast for example to apparatus utilising a ball valve type of flow mechanism. Also, the construction of the device is such as to enable continuance of gas supply by minimising the risk of the concealment member becoming snagged, while enabling ongoing gas supply even if the concealment member does become snagged.

While this invention has been described in connection with specific embodiments thereof, it will be understood that it is capable of further modification(s). The present invention is intended to cover any variations, uses or adaptations of the invention following in general, the principles of the invention and including such departures from the present disclosure as come within known or customary practice within the art to which the invention pertains and as may be applied to the essential features hereinbefore set forth.

As the present invention may be embodied in several forms without departing from the spirit of the essential characteristics of the invention, it should be understood that the above described embodiments are not to limit the present invention unless otherwise specified, but rather should be construed broadly within the spirit and scope of the invention as defined in the attached claims. Various modifications and equivalent arrangements are intended to be included within the scope of the invention as defined by the appended claims. Therefore, the specific embodiments are to be understood to be illustrative of the many ways in which the principles of the present invention may be practiced.

Where the terms "comprise", "comprises", "comprised" or "comprising" are used in this specification, they are to be interpreted as specifying the presence of the stated features, integers, steps or components referred to, but not to preclude the presence or addition of one or more other features, integers, steps, components to be grouped therewith.

## Claims

1. A gas flow indicator device (10) including an elongate housing (12) that has a peripheral sidewall (14) that extends between first and second opposite ends and that defines a gas flow chamber (20) through which gas is able to flow from an inlet port (22) at a first end (16) to an outlet port (24) at a second end (18); wherein, within the chamber (20), the device (10) has a gas flow indicator member (26) that extends from adjacent to the first end (16) of the housing over part of the length of the chamber (20) towards the second end (18); the indicator member (26) is of annular form such that gas flowing through the chamber (20) from the inlet port (22) to the outlet port (24) enters the chamber (20) along a bore (28) that extends through the indicator member(26); the indicator member (26) is spaced from an inner surface (30) of the sidewall (14) of the housing (12) to define, with that inner surface (30), an annular space (32) that forms part of the chamber (20); the device (10) further includes a concealment member (34) that is movable from the first end (16) of the housing (12) in response to a sufficient pressure generated by gas flow from the inlet port (22) to the outlet port (24), against the action of a biasing member (36) acting to bias the concealment member (34) to the first end (16); the concealment member (34) has an annular peripheral skirt (38) that, under the bias of the biasing member on the concealment member (34), is receivable into the annular space, such that one of opposite ends of the skirt (38) is able to seal against an annular surface of the housing (12) at or adjacent to an end wall (44) of the housing (12) at the first end (16) of the housing (12) ; and the concealment member (34) also includes, at the other of the opposite ends of the skirt (38), a transverse wall (48) that defines an opening (50) through which the flow of gas from the inlet port (22) to the outlet port (24) is able to pass, with the opening (50) providing resistance to such flow; wherein the arrangement is such that the one of opposite ends of the skirt (38) is able to be held in sealing engagement with the annular surface at or adjacent to the end wall (44) of the housing (12) whereby the indicator member (26) is concealed from view, through a laterally adjacent viewing window portion (52) of the housing (12), when there is no gas flow or a gas flow rate generating less than the sufficient pressure but such that, with increasing gas flow rate the sufficient pressure is achieved and the bias of the biasing member is thereby overcome to enable the concealment member (34) to move towards the second end of the housing and expose the indicator member (26) to view through the viewing window portion (52) of the housing (12) and provide a visual indication indicative of gas flow.

2. The gas flow indicator device (10) of claim 1, wherein movement of the concealment member to expose the indicator member (26) moves the one of opposite ends of the skirt (38) of the concealment member (34) from the annular surface at or adjacent to the end wall (44) of the housing (12), such as thereby to enable a secondary gas flow to the outlet that passes around the one of opposite ends of the skirt (38) between the concealment member (34) and the housing (12).

3. The gas flow indicator device (10) of claim 1 or claim 2, wherein the biasing member either: acts in compression in biasing the concealment member (34) to the first end (16) of the housing, such as by the biasing member (36) being compressed between, such as compressed by, the concealment member (34) and the second end (18) so as to provide the required bias in seeking to expand; or, acts in tension in biasing the concealment member (34) to the first end (16) of the housing (12), such as by the biasing member (36) being within the concealment member (34), and tensioned by being expanded between, such as by being connected in relation to, each of the transverse wall (48) of the concealment member (36) and the first end (16) of the housing (12) so as to provide the bias in seeking to contract.

4. The gas flow indicator device (10) of claim 3, wherein the biasing member (36) either: comprises a coil spring acting between the concealment member (34) and the second end (18) of the housing (12), and wherein compression of the spring can limit the extent to which the concealment member (36) can move towards the second end (18) of the housing (12) simply as a consequence of the presence of the compressed spring; or, comprises a coil spring within the concealment member (34), with the arrangement such that the concealment member (34) is able to move so as to contact the end wall at the second end (18) of the housing (12), or such that contact of the concealment member (34) with the second end (18) of the housing (12) is precluded by the spring reaching its elastic limit.

5. The gas flow indicator device (10) of any one of the preceding claims, wherein the concealment member (34) is able to move, between a first position in which the indicator member (26) is concealed and a second position in which the indicator member (26) is sufficiently exposed, or fully or substantially fully exposed, over a distance enabling a clear visual indication of gas flow through the device (10), with the arrangement such that, when so exposed, the indicator member (26) can readily be seen through the viewing window portion (52) of the sidewall (14) of the housing (12) by a person in close proximity, such as adjacent to the device (10) and preferably also from a considerable distance such as from five to ten meters, or further, from the device (10).

6. The gas flow indicator device (10) of claim 5, wherein the viewing window portion (52) is at least translucent, but preferably also is sufficiently transparent to facilitate viewing of the indicator member (26), when exposed, and most preferably is of high transparency, and wherein the viewing window portion (52) extends around the full circumferential extent of the sidewall (14) of the housing (12) adjacent to the first end (16), or comprises two or more regions spaced circumferentially around that wall of the housing adjacent to the first end (16).

7. The gas flow indicator device (10) of claim 5 or claim 6, wherein the viewing window portion (52) is of a high-transparency plastics or glass material, such as of a material also having a high level of clarity, and wherein the material of which the remainder of the housing (12) is made may be transparent, translucent or opaque, as required.

8. The gas flow indicator device (10) of any one of the preceding claims, wherein the housing (12) is circular in cross-sections perpendicular to the direction of spacing between the first (16) and second ends (18), with the housing (12) having an inner surface (30) that is substantially cylindrical and of substantially constant circular transverse cross-section between the first (16) and second ends (18), and with the sidewall (14) also having an outer surface (54) that is substantially cylindrical and of substantially constant circular cross-section.

9. The gas flow indicator device (10) of claim 8, wherein the housing (12) has an outer surface (54) of the sidewall (14) that departs from a cylindrical form, at least along an initial section of its longitudinal extent from adjacent to the first end (16), with that initial section extending around the gas flow indicator member (26), and wherein the outer surface (54) of the sidewall (14), over that initial section of its longitudinal extent, smoothly increases in diameter to a maximum in a direction away from the first end (16) towards the second end (18), and thereafter smoothly decreases in diameter substantially to the diameter at the first end (16), so as to have a convex longitudinal form.

10. The gas flow indicator device (10) of any one of the preceding claims, wherein at the first end (16) of the housing (12), the inlet port (22) is defined by a spigot (56) that projects from the first end (16) of the housing (12) in a direction away from the second end (18), and that enables connection of a gas supply conduit (C) to the housing (12).

11. The gas flow indicator device (10) of claim 10, wherein the spigot (56) has an extension that projects into the interior of the housing (12) to provide a hub (58) on or around which the gas flow indicator member (26) is mounted, with the indicator member (26) preferably having an axial extent that is substantially the same as, or slightly greater than, the length of the hub (56), with the indicator member (26) being a firm friction fit on the hub (58) or by the indicator member (26) being bonded or welded onto the hub (58), or by the indicator member (26) having an end, adjacent to the end wall (44) at the first end (16) of the housing (12), by which the indicator member (26) is bonded or welded to the end wall (44) or the indicator member (26) has, at its end remote from the first end (16) of the housing (12), a flange that overlaps the corresponding end of the hub (58), with the flange bonded or welded to that end of the hub (58).

12. The gas flow indicator device (10) of claim 10, wherein the indicator member (26) is bonded or welded to an end wall (44) of the housing (12) at the first end (16), or mechanically engaged with that end wall such a by screw-threaded engagement or a snap fit in or around the inlet port (22).

13. The gas flow indicator device (10) of any one of the preceding claims, wherein the skirt (38) of the concealment member (34) has an outer surface (60) that is spaced from the inner surface (30) of the sidewall (14) of the housing (12), with a spacing between the surfaces throughout the extent of movement of the concealment member (34) longitudinally within the housing (12), and wherein the gas flow indicator device (10) further includes guide members that maintain the concealment member (34) in a substantially co-axial relationship with the housing (12), such guide members preferably comprising a plurality of circumferentially spaced projections that stand proud, or project inwardly, of the inner surface (30) of the housing (12) so as to be contactable with the outer surface (60) of the skirt (38) of the concealment member (34), or the converse, or a plurality of longitudinal ribs on the inner surface (30) of the sidewall (14) of the housing (12) or the outer surface (60) of the concealment member (34).

14. The gas flow indicator device (10) of any one of the preceding claims, wherein at the second end (18), the housing (12) has an end wall (44) that defines the outlet port (24), with the end wall (44) preferably being separable from the sidewall (14) of the housing (12), such as by the end wall (44) comprising an annular peripheral rim (66) that is engaged with the sidewall (14) of the housing (12) around the second end (18), a central hub portion (68) and a plurality of circumferentially spaced connectors (70) that extend between the rim (66) and the hub.

15. A gas delivery device, gas delivery system or gas supply conduit including the gas flow indicator device (10) of any one of the preceding claims.

## Patentansprüche

1. Eine Gas-Durchflussanzeigevorrichtung (10) mit einem länglichen Gehäuse (12), das eine Umfangsseitenwand (14) aufweist, die sich zwischen einem ersten und einem zweiten gegenüberliegenden Ende erstreckt und eine Gasdurchflusskammer (20) definiert, durch die Gas von einer Einlassöffnung (22) an einem ersten Ende (16) zu einer Auslassöffnung (24) an einem zweiten Ende (18) strömen kann; wobei die Vorrichtung (10) innerhalb der Kammer (20) ein Gasströmungsanzeigerelement (26) aufweist, das sich von angrenzend an das erste Ende (16) des Gehäuses über einen Teil der Länge der Kammer (20) zum zweiten Ende (18) erstreckt; das Anzeigeelement (26) ringförmig ist, so dass Gas, das durch die Kammer (20) von der Einlassöffnung (22) zur Auslassöffnung (24) strömt, entlang einer Bohrung (28), die sich durch das Anzeigeelement (26) erstreckt, in die Kammer (20) eintritt; das Anzeigeelement (26) ist von einer Innenfläche (30) der Seitenwand (14) des Gehäuses (12) beabstandet, um mit dieser Innenfläche (30) einen ringförmigen Raum (32) zu definieren, der Teil der Kammer (20) bildet; die Vorrichtung (10) ferner ein Abdeckelement (34) beinhaltet, das vom ersten Ende (16) des Gehäuses (12) als Reaktion auf einen ausreichenden Druck, der durch den Gasstrom von der Einlassöffnung (22) zur Auslassöffnung (24) erzeugt wird, gegen die Wirkung eines Vorspannelements (36) beweglich ist, das das Abdeckelement (34) zum ersten Ende (16) vorspannt; das Abdeckelement (34) eine ringförmige Umfangsschürze (38) aufweist, die unter der Vorspannung des Vorspannelements am Abdeckelement (34) in den Ringraum aufnehmbar ist, so dass eines der gegenüberliegenden Enden der Schürze (38) gegen eine ringförmige Oberfläche des Gehäuses (12) an oder angrenzend an eine Stirnwand (44) des Gehäuses (12) am ersten Ende (16) des Gehäuses (12) abdichten kann ; und das Abdeckelement (34) auch am anderen der gegenüberliegenden Enden des Schirms (38) eine Querwand (48) aufweist, die eine Öffnung (50) definiert, durch die der Gasstrom von der Einlassöffnung (22) zur Auslassöffnung (24) hindurchtreten kann, wobei die Öffnung (50) einen Widerstand gegen diesen Strom bildet; wobei die Anordnung derart ist, dass das eine der gegenüberliegenden Enden des Schirms (38) in dichtendem Eingriff mit der ringförmigen Oberfläche an oder angrenzend an die Stirnwand (44) des Gehäuses (12) gehalten werden kann, wodurch das Anzeigeelement (26) durch einen seitlich angrenzenden Sichtfensterabschnitt (52) des Gehäuses (12) verborgen ist, wenn kein Gasfluss oder eine Gasdurchflussmenge vorliegt, die weniger als den ausreichenden Druck erzeugt, aber derart, dass, mit zunehmender Gasdurchflussmenge wird der ausreichende Druck erreicht und die Vorspannung des Vorspannelements überwunden, um es dem Abdeckelement (34) zu ermöglichen, sich in Richtung des zweiten Endes des Gehäuses zu bewegen und das Anzeigeelement (26) freizulegen, um durch den Sichtfensterabschnitt (52) des Gehäuses (12) zu sehen und eine visuelle Anzeige zu liefern, die den Gasfluss anzeigt.

2. Gas-Durchflussanzeigevorrichtung (10) nach Anspruch 1, wobei die Bewegung des Abdeckelements, um das Anzeigeelement (26) freizulegen, das eine der gegenüberliegenden Enden des Randes (38) des Abdeckelements (34) von der ringförmigen Oberfläche an oder angrenzend an die Stirnwand (44) des Gehäuses (12) bewegt, um dadurch einen Sekundärgasstrom zum Auslass zu ermöglichen, der um das eine der gegenüberliegenden Enden des Randes (38) zwischen dem Abdeckelement (34) und dem Gehäuse (12) verläuft.

3. Die Gasflussanzeigevorrichtung (10) nach Anspruch 1 oder Anspruch 2, wobei das Vorspannelement (36) entweder: wirkt komprimiert beim Vorspannen des Abdeckelements (34) zum ersten Ende (16) des Gehäuses, wie beispielsweise durch das Vorspannelement (36), das zwischen dem Abdeckelement (34) und dem zweiten Ende (18) komprimiert wird, um die erforderliche Vorspannung beim Streben nach Expansion zu erzeugen; oder spannungsführend wirkt, wenn es das Verdeckungselement (34) bis zum ersten Ende (16) des Gehäuses (12) vorspannt, wie beispielsweise durch das Vorspannelement (36), das sich innerhalb des Verdeckungselements (34) befindet, und durch Ausdehnung zwischen jeder der Querwände (48) des Verdeckungselements (36) und dem ersten Ende (16) des Gehäuses (12) gespannt wird, um die Vorspannung bei der Suche nach einer Kontraktion zu erzeugen.

4. Die Gasflussanzeigevorrichtung (10) nach Anspruch 3, wobei das Vorspannelement (36) entweder: eine Schraubenfeder umfasst, die zwischen dem Abdeckelement (34) und dem zweiten Ende (18) des Gehäuses (12) wirkt, und wobei die Kompression der Feder das Ausmaß begrenzen kann, in dem sich das Abdeckelement (36) in Richtung des zweiten Endes (18) des Gehäuses (12) allein durch das Vorhandensein der komprimierten Feder bewegen kann; oder eine Schraubenfeder innerhalb des Abdeckelements (34) umfasst, wobei die Anordnung so ist, dass sich das Abdeckelement (34) so bewegen kann, dass es die Stirnwand am zweiten Ende (18) des Gehäuses (12) berührt, oder dass ein Kontakt des Abdeckelements (34) mit dem zweiten Ende (18) des Gehäuses (12) ausgeschlossen ist, wenn die Feder ihre elastische Grenze erreicht.

5. Gas-Durchflussanzeigevorrichtung (10) eines der vorhergehenden Ansprüche, wobei das Abdeckelement (34) in der Lage ist, sich zwischen einer ersten Position, in der das Anzeigeelement (26) verborgen ist, und einer zweiten Position, in der das Anzeigeelement (26) ausreichend oder vollständig oder im Wesentlichen vollständig freigelegt ist, über einen Abstand zu bewegen, der eine klare visuelle Anzeige des Gasstroms durch die Vorrichtung (10) ermöglicht, mit der Anordnung, so dass das Anzeigeelement (26) bei solcher Freilegung durch den Sichtfensterabschnitt (52) der Seitenwand (14) des Gehäuses (12) von einer Person in unmittelbarer Nähe, wie beispielsweise benachbart zur Vorrichtung (10), und vorzugsweise auch aus einer größeren Entfernung, wie beispielsweise aus fünf bis zehn Metern oder weiter von der Vorrichtung (10), gut sichtbar ist.

6. Gas-Durchflussanzeigevorrichtung (10) nach Anspruch 5, wobei der Sichtfensterabschnitt (52) zumindest lichtdurchlässig, aber vorzugsweise auch ausreichend transparent ist, um das Betrachten des Anzeigeelements (26) bei Freilegung zu erleichtern, und höchstmöglicherweise von hoher Transparenz ist, und wobei sich der Sichtfensterabschnitt (52) um die volle Umfangsausdehnung der Seitenwand (14) des Gehäuses (12) angrenzend an das erste Ende (16) erstreckt oder zwei oder mehr Bereiche umfasst, die in Umfangsrichtung um diese Wand des Gehäuses angrenzend an das erste Ende (16) angeordnet sind.

7. Gas-Durchflussanzeigevorrichtung (10) nach Anspruch 5 oder Anspruch 6, wobei der Sichtfensterabschnitt (52) aus einem hochtransparenten Kunststoff- oder Glasmaterial besteht, wie beispielsweise aus einem Material, das ebenfalls ein hohes Maß an Klarheit aufweist, und wobei das Material, aus dem der Rest des Gehäuses (12) besteht, je nach Bedarf transparent, lichtdurchlässig oder opak sein kann.

8. Gas-Durchflussanzeigevorrichtung (10) nach einem der vorstehenden Ansprüche, wobei das Gehäuse (12) kreisförmig in Querschnitten senkrecht zur Abstandsrichtung zwischen dem ersten (16) und dem zweiten Ende (18) ist, wobei das Gehäuse (12) eine Innenfläche (30) aufweist, die im Wesentlichen zylindrisch ist und einen im Wesentlichen konstanten kreisförmigen Querschnitt zwischen dem ersten (16) und dem zweiten Ende (18) aufweist, und wobei die Seitenwand1 (14) auch eine Außenfläche (54) aufweist, die im Wesentlichen zylindrisch und einen im Wesentlichen konstanten kreisförmigen Querschnitt aufweist.

9. Gas-Durchflussanzeigevorrichtung (10) nach Anspruch 8, wobei das Gehäuse (12) eine Außenfläche (54) der Seitenwand (14) aufweist, die von einer zylindrischen Form abweicht, zumindest entlang eines anfänglichen Abschnitts ihrer Längserstreckung von benachbart zum ersten Ende (16), wobei sich dieser anfängliche Abschnitt um das Gasströmungsanzeigeelement (26) erstreckt, und wobei die Außenfläche (54) der Seitenwand (14) über diesen anfänglichen Abschnitt ihrer Längserstreckung den Durchmesser bis zu einem Maximum in einer Richtung weg vom ersten Ende (16) zum zweiten Ende (18) sanft vergrößert und danach den Durchmesser im Wesentlichen auf den Durchmesser am ersten Ende (16) reduziert, um eine konvexe Längsform zu haben.

10. Gas-Durchflussanzeigevorrichtung (10) nach einem der vorhergehenden Ansprüche, wobei am ersten Ende (16) des Gehäuses (12) die Einlassöffnung (22) durch einen Stutzen (56) definiert ist, der vom ersten Ende (16) des Gehäuses (12) in eine vom zweiten Ende (18) entfernte Richtung ragt und den Anschluss einer Gasversorgungsleitung (C) an das Gehäuse (12) ermöglicht.

11. Gas-Durchflussanzeigevorrichtung (10) nach Anspruch 10, wobei der Stutzen (56) eine Verlängerung aufweist, die in das Innere des Gehäuses (12) ragt, um eine Nabe (58) (Englisch: "a hub") bereitzustellen, an oder um die das Gasströmungsanzeigerelement (26) montiert ist, wobei das Anzeigeelement (26) vorzugsweise eine axiale Ausdehnung aufweist, die im Wesentlichen gleich oder etwas größer als die Länge der Nabe (56) ist, wobei das Anzeigeelement (26) ein fester Reibsitz auf der Nabe (58) ist oder indem das Anzeigeelement (26) auf die Nabe (58) geklebt oder geschweißt ist, oder durch das Anzeigeelement (26) mit einem Ende, das an die Stirnwand (44) am ersten Ende (16) des Gehäuses (12) angrenzt, durch das das Anzeigeelement (26) mit der Stirnwand (44) verklebt oder verschweißt ist oder das Anzeigeelement (26) an seinem vom ersten Ende (16) des Gehäuses (12) entfernten Ende einen Flansch aufweist, der das entsprechende Ende der Nabe (58) überlappt, wobei der Flansch mit diesem Ende der Nabe (58) verklebt oder verschweißt ist.

12. Gas-Durchflussanzeigevorrichtung (10) nach Anspruch 10, wobei das Anzeigeelement (26) mit einer Stirnwand (44) des Gehäuses (12) am ersten Ende (16) verklebt oder verschweißt ist oder mechanisch mit dieser Stirnwand in Eingriff steht, beispielsweise durch Schraubgewindeeingriff oder Schnappsitz in oder um die Einlassöffnung (22).

13. Gas-Durchflussanzeigevorrichtung (10) nach einem der vorstehenden Ansprüche, wobei die Schürze (38) des Abdeckelements (34) eine Außenfläche (60) aufweist, die von der Innenfläche (30) der Seitenwand (14) des Gehäuses (12) beabstandet ist, mit einem Abstand zwischen den Oberflächen über den gesamten Bewegungsumfang des Abdeckelements (34) in Längsrichtung innerhalb des Gehäuses (12), und wobei die Gas-Durchflussanzeigevorrichtung (10) ferner Führungselemente beinhaltet, die das Abdeckelement (34) in einer im Wesentlichen achsialen Beziehung mit dem Gehäuse (12) halten, solche Führungselemente, die vorzugsweise eine Vielzahl von in Umfangsrichtung beabstandeten Vorsprüngen umfassen, die herausstehen oder auf die Innenfläche (30) des Gehäuses (12) vorstehen, sodass sie mit der Außenfläche (60) des Randes (38) des Abdeckelements (34) oder dem Umkehrschluss oder einer Vielzahl von Längsrippen auf der Innenfläche (30) der Seitenwand (14) des Gehäuses (12) oder der Außenfläche (60) des Abdeckelements (34) kontaktierbar sind.

14. Gas-Durchflussanzeigevorrichtung (10) nach einem der vorstehenden Ansprüche, wobei das Gehäuse (12) am zweiten Ende (18) eine Stirnwand (44) aufweist, die die Austrittsöffnung (24) definiert, wobei die Stirnwand (44) vorzugsweise von der Seitenwand (14) des Gehäuses (12) trennbar ist, wie beispielsweise durch die Stirnwand (44), die einen ringförmigen Umfangsrand (66) umfasst, der mit der Seitenwand (14) des Gehäuses (12) um das zweite Ende (18) herum in Eingriff steht, einen zentralen Nabenabschnitt (68) und eine Vielzahl von in Umfangsrichtung beabstandeten Verbindern (70), die sich zwischen dem Rand (66) und der Nabe erstrecken.

15. Gaszufuhrvorrichtung, Gaszufuhrsystem oder Gaszufuhrleitung mit der Gasdurchflussanzeigevorrichtung (10) eines der vorhergehenden Ansprüche.

## Revendications

1. Dispositif indicateur de débit de gas (10) comprenant un boîtier allongé (12) qui présente une paroi latérale périphérique (14) qui s'étend entre une première et une deuxième extrémités opposées et qui définit une chambre d'écoulement de gaz (20) à travers laquelle le gaz peut s'écouler d'un orifice d'entrée (22) à une première extrémité (16) à un orifice de sortie (24) à une deuxième extrémité (18) ; dans laquelle, à l'intérieur de la chambre (20), le dispositif (10) comporte un élément indicateur de débit de gaz (26) qui s'étend du voisinage de la première extrémité (16) du boîtier sur une partie de la longueur de la chambre (20) vers la seconde extrémité (18) ; l'élément indicateur (26) est de forme annulaire de telle sorte que le gaz s'écoulant à travers la chambre (20) depuis l'orifice d'entrée (22) vers l'orifice de sortie (24) entre dans la chambre (20) le long d'un alésage (28) qui traverse l'élément indicateur (26) ; l'élément indicateur (26) est séparé d'une surface intérieure (30) de la paroi latérale (14) du logement (12) pour définir, avec cette surface intérieure (30), un espace annulaire (32) qui fait partie de la chambre (20) ; le dispositif (10) comprend en outre un élément d'occultation (34) qui est mobile depuis la première extrémité (16) du boîtier (12) en réponse à une pression suffisante générée par un écoulement de gaz depuis l'orifice d'entrée (22) vers l'orifice de sortie (24), contre l'action d'un élément de polarisation (36) agissant pour polariser l'élément d'occultation (34) vers la première extrémité (16) ; l'élément de dissimulation (34) comporte une jupe périphérique annulaire (38) qui, sous la sollicitation de l'élément de sollicitation sur l'élément de dissimulation (34), est recevable dans l'espace annulaire, de sorte que l'une des extrémités opposées de la jupe (38) est capable de s'appliquer contre une surface annulaire du boîtier (12) sur ou adjacente à une paroi frontale (44) du boîtier (12) à la première extrémité (16) du boîtier (12), ; et l'élément de dissimulation (34) comprend également, à l'autre extrémité opposée de la jupe (38), une paroi transversale (48) qui définit une ouverture (50) à travers laquelle le flux de gaz de l'orifice d'entrée (22) à l'orifice de sortie (24) peut passer, l'ouverture (50) fournissant une résistance à ce flux ; dans lequel l'agencement est tel que l'une des extrémités opposées de la jupe (38) peut être maintenue en contact étanche avec la surface annulaire au niveau de la paroi d'extrémité (44) du boîtier (12) ou à proximité de celle-ci, l'élément indicateur (26) étant dissimulé à travers une partie (52) du boîtier (12) de fenêtre de visualisation adjacente sur le côté, lorsque aucun débit de gaz ou un débit de gaz ne génère moins que la pression suffisante mais tel quel, avec un débit de gaz croissant, la pression suffisante est obtenue et la polarisation de l'élément de polarisation est ainsi surmontée pour permettre à l'élément d'occultation (34) de se déplacer vers la deuxième extrémité du boîtier et exposer l'élément indicateur (26) pour voir à travers la partie de fenêtre de visualisation (52) du boîtier (12) et fournir une indication visuelle indiquant le débit du gaz.

2. Dispositif indicateur de débit de gaz (10) selon la revendication 1, dans lequel le mouvement de l'élément de dissimulation pour exposer l'élément indicateur (26) déplace l'une des extrémités opposées de la jupe (38) de l'élément de dissimulation (34) depuis la surface annulaire au niveau ou à proximité de la paroi d'extrémité (44) du boîtier (12), de manière à permettre un débit gazeux secondaire vers la sortie qui passe autour de l'une des extrémités opposées de la jupe (38) entre l'élément de dissimulation (34) et le boîtier (12), ce afin d'assurer ainsi le passage d'un débit gazeux secondaire à l'évacuation qui se produit entre l'une des extrémités opposées de la jupe (38).

3. Dispositif indicateur de débit de gaz (10) selon la revendication 1 ou la revendication 2, dans lequel l'élément de polarisation soit : agit en compression en sollicitant l'élément de dissimulation (34) jusqu'à la première extrémité (16) du boîtier, par exemple en comprimant l'élément de sollicitation (36) entre l'élément de dissimulation (34) et la seconde extrémité (18), de manière à fournir la sollicitation nécessaire pour chercher à se dilater ; ou, agit en tension en sollicitant l'élément d'occultation (34) jusqu'à la première extrémité (16) du boîtier (12), tel que par le fait que l'élément de sollicitation (36) se trouve dans l'élément d'occultation (34), et en tension en étant dilaté entre, tel que par être relié par rapport à, chacune de la paroi transversale (48) de l'élément d'occultation (36) et la première extrémité (16) du boîtier (12) afin de provoquer la contrainte en recherchant à contracter.

4. Dispositif indicateur de débit de gaz (10) selon la revendication 3, dans lequel l'élément de précontrainte (36) soit : comprend un ressort hélicoïdal agissant entre l'élément d'occultation (34) et la seconde extrémité (18) du boîtier (12), et dans lequel la compression du ressort peut limiter la mesure dans laquelle l'élément d'occultation (36) peut se déplacer vers la seconde extrémité (18) du boîtier (12) simplement en raison de la présence du ressort comprimé ; ou, comprend un ressort hélicoïdal à l'intérieur de l'élément d'occultation (34), l'élément d'occultation (34) étant disposé de telle sorte que l'élément d'occultation (34) puisse se déplacer de manière à venir en contact avec la paroi d'extrémité à la deuxième extrémité (18) du boîtier (12), ou que le contact de l'élément d'occultation (34) avec la deuxième extrémité (18) du boîtier (12) est interdit lorsque le ressort atteint sa limite élastique.

5. Dispositif indicateur de débit de gaz (10) de l'une quelconque des revendications précédentes, dans lequel l'élément de dissimulation (34) est capable de se déplacer, entre une première position dans laquelle l'élément indicateur (26) est dissimulé et une seconde position dans laquelle l'élément indicateur (26) est suffisamment exposé, ou entièrement ou largement entièrement exposé, sur une distance permettant une indication visuelle claire du débit gazeux traversant le dispositif (10), avec l'agencement de telle sorte que, lorsqu'il est ainsi exposé, l'élément indicateur (26) peut être facilement vu à travers la partie de fenêtre de visualisation (52) de la paroi latérale (14) du boîtier (12) par une personne à proximité immédiate, telle que adjacente au dispositif (10) et de préférence également à une distance considérable telle que de cinq à dix mètres, ou plus, du dispositif (10).

6. Dispositif indicateur d'écoulement de gaz (10) selon la revendication 5, dans lequel la partie de fenêtre de visualisation (52) est au moins translucide, mais de préférence suffisamment transparente pour faciliter la visualisation de l'élément indicateur (26), lorsqu'elle est exposée, et de préférence de haute transparence, et dans lequel la partie de fenêtre de visualisation (52) s'étend autour de l'étendue périphérique totale de la paroi latérale (14) du boîtier (12) adjacente à la première extrémité (16) ou comprend deux régions ou plus, espacées circonféremment, entourant la paroi du boîtier adjacente à la première extrémité (16).

7. Dispositif indicateur de débit de gaz (10) selon la revendication 5 ou la revendication 6, dans lequel la partie de fenêtre de visualisation (52) est en matière plastique ou en verre à haute transparence, telle qu'un matériau présentant également un haut niveau de clarté, et dans lequel le matériau dont est constitué le reste du boîtier (12) peut être transparent, translucide ou opaque, selon les besoins.

8. Dispositif indicateur de débit de gaz (10) de l'une quelconque des revendications précédentes, dans lequel le boîtier (12) a une section transversale circulaire perpendiculaire à la direction d'espacement entre la première (16) et la seconde extrémité (18), le boîtier (12) ayant une surface intérieure (30) qui est largement cylindrique et de section transversale circulaire largement constante entre la première (16) et la seconde extrémité (18), et le côté (14) ayant également une surface extérieure (54) qui est largement cylindrique et de section circulaire largement constante.

9. Dispositif indicateur de débit de gaz (10) selon la revendication 8, **caractérisé en ce que** le boîtier (12) présente une surface extérieure (54) de la paroi latérale (14) qui s'écarte d'une forme cylindrique, au moins le long d'une partie initiale de son étendue longitudinale, par rapport au voisinage de la première extrémité (16), cette partie initiale étant disposée autour du corps indicateur de débit de gaz (26), et dans laquelle la surface extérieure (54) de la paroi latérale (14), sur cette section initiale de son extension longitudinale, augmente doucement son diamètre jusqu'à un maximum dans une direction s'éloignant de la première extrémité (16) vers la seconde extrémité (18), et diminue ensuite doucement son diamètre largement au diamètre à la première extrémité (16), de manière à avoir une forme longitudinale convexe.

10. Dispositif indicateur de débit de gaz (10) selon l'une quelconque des revendications précédentes, dans lequel, à la première extrémité (16) du boîtier (12), l'orifice d'entrée (22) est défini par un cône (56) qui dépasse de la première extrémité (16) du boîtier (12) dans une direction opposée à la seconde extrémité (18), et qui permet la connexion d'une conduite de gaz (C) au boîtier (12).

11. Dispositif indicateur de débit de gaz (10) selon la revendication 10, dans lequel cône (56) présente une extension qui fait saillie à l'intérieur du boîtier (12) pour former un moyeu (58) sur ou autour duquel l'élément indicateur de débit de gaz (26) est monté, l'élément indicateur (26) ayant de préférence une étendue axiale qui est largement identique ou légèrement supérieure à la longueur du moyeu (56), l'élément indicateur (26) étant un ajustement ferme sur le moyeu (58) ou l'élément indicateur (26) étant lié ou soudé sur le moyeu (58), ou par l'élément indicateur (26) ayant une extrémité, adjacente à la paroi d'extrémité (44) à la première extrémité (16) du boîtier (12), par laquelle l'élément indicateur (26) est collé ou soudé à la paroi d'extrémité (44) ou l'élément indicateur (26) a, à son extrémité opposée à la première extrémité (16) du boîtier (12), une bride qui recouvre l'extrémité correspondante du moyeu (58), la bride collée ou soudée à cette extrémité du moyeu (58).

12. Dispositif indicateur de débit de gaz (10) selon la revendication 10, dans lequel l'élément indicateur (26) est collé ou soudé à une paroi d'extrémité (44) du boîtier (12) à la première extrémité (16), ou en prise mécanique avec cette paroi d'extrémité, par engagement par vissage ou encliquetage dans ou autour de l'entrée (22).

13. Dispositif indicateur d'écoulement de gaz (10) de l'une quelconque des revendications précédentes, dans lequel la jupe (38) de l'élément de dissimulation (34) présente une surface extérieure (60) qui est espacée de la surface intérieure (30) de la paroi latérale (14) du boîtier (12), avec un espace entre les surfaces dans l'étendue du mouvement longitudinal de l'élément de dissimulation (34) dans l'enceinte (12) et dans lequel le dispositif indicateur de flux de gaz (10) comprend en outre des éléments de guidage qui maintiennent l'élément de dissimulation (34) largement dans une position axe au boîtier (12), de tels éléments de guidage comprenant de préférence une pluralité de saillies espacées circonférentiellement qui s'étendant, ou font saillie vers l'intérieur, de la surface intérieure (30) du boîtier (12) de manière à pouvoir entrer en contact avec la surface extérieure (60) de la jupe (38) de l'élément de recouvrement (34), ou la contre-couche, ou une pluralité de nervures longitudinales sur la surface intérieure (30) de la paroi latérale (14) du boîtier (12) ou la surface extérieure (60) de l'élément de recouvrement (34).

14. Dispositif indicateur de débit de gaz (10) de l'une quelconque des revendications précédentes, dans lequel, à la deuxième extrémité (18), le boîtier (12) présente une paroi d'extrémité (44) qui définit l'orifice de sortie (24), la paroi d'extrémité (44) étant de préférence séparable de la paroi latérale (14) du boîtier (12), tel que par la paroi d'extrémité (44) comprenant une jante périphérique annulaire (66) qui est en prise avec la paroi latérale (14) du boîtier (12) autour de la seconde extrémité (18), une partie centrale de moyeu (68) et une pluralité de connecteurs (70) espacés en circonférence qui s'étendent entre la jante (66) et le moyeu.

15. Dispositif d'alimentation en gaz, système d'alimentation en gaz ou conduite d'alimentation en gaz, y compris le dispositif indicateur de débit de gaz (10) de l'une quelconque des revendications précédentes.
